# EUROPEAN PATENT APPLICATION

(11) **EP 4 678 648 A1**
(43) Date of publication of application: **14.01.2026**
(21) Application number: 24767364.3
(22) Date of filing: 04.03.2024
(51) Int. Cl.: C07K 1/13, C07K 14/31, C07K 16/00, A61K 47/68

(54) **SYNTHESIS METHOD OF ANTIBODY-DRUG CONJUGATES USING PROXIMITY EFFECT-BASED SITE-SELECTIVE ANTIBODY LABELING**

(30) Priority: 03.03.2023 KR 20230028269; 16.11.2023 KR 20230159105
(71) Applicant: Sogang University Research Business Development Foundation, Seoul 04107 (KR); Research & Business Foundation SungKyunKwan University, Gyeonggi-do 16419 (KR)
(72) Inventor: LEE, Hyun Soo, Goyang-si Gyeonggi-do 10551 (KR); KIM, Sooin, Seoul 07021 (KR); KWEON, Yongseok, Seoul 06767 (KR)
(74) Representative: Algemeen Octrooi- en Merkenbureau B.V.
(86) International application number: PCT/KR2024/002726
(87) International publication number: WO 2024/186075

(57) **Abstract**

The present invention relates to a method for the synthesis of an antibody-drug conjugate using proximity effect-based site-selective antibody labeling. Provided according to the present invention may be a method for the synthesis of an antibody-drug conjugate by site-selectively introducing a drug into an antibody using a pyridinium oxime derivative (labeling mediator protein) introduced into a binding protein containing a non-standard amino acid.

## Description

### [Technical Field]

The present disclosure relates to a synthesis method of an antibody-drug conjugate using proximity effect-based site-selective antibody labeling.

### [Background Art]

Antibody-drug conjugates (ADCs) are one of the fastest-growing classes among various novel anticancer drugs, and comprise an antibody conjugated to a cytotoxic drug (payload). Here, the antibody serves to recognize proteins specifically expressed on cancer cells (targeting), and the cytotoxic drug exerts toxicity within the recognized cancer cells. The design is intended to minimize toxicity and side effects on normal cells, which are observed with small-molecule anticancer drugs.

The development of ADCs for actual therapeutic applications requires consideration of numerous factors. Specifically, an appropriate antigen expressed on the target cancer cells, an antibody that selectively recognizes the antigen, and a drug exhibiting cytotoxicity are important factors. Furthermore, in order to conjugate the antibody and the drug, appropriate conjugation technologies and an appropriate linker connecting the antibody and the drug should also be taken into account as important factors.

In addition, the properties of ADCs are significantly influenced not only by the antibody, drug, and linker, but also by the site, number, and uniformity of drug incorporation. Consequently, the properties of ADCs are determined by the antibody-drug conjugation technology, and the development of novel antibody-drug conjugation technologies provides a broad platform technology applicable across various ADC fields, independent of the nature of the cancer cells.

Currently available conjugation technologies for drug incorporation into antibodies include: (1) chemical incorporation method utilizing lysine or cysteine, and (2) site-specific incorporation methods utilizing an enzyme. First, among chemical methods, the method utilizing lysine on the antibody surface has the greatest advantage in that it allows the use of produced antibodies without modification. However, it suffers from a lack of site selectivity and exhibits low homogeneity in ADCs. Such lack of site specificity and heterogeneity in ADCs may lead to reduced antibody binding affinity and difficulties in quality control during the production process. In the other hand, the method utilizing cysteine enable the synthesis of relatively homogeneous ADCs; however, it still exhibits low homogeneity and has the drawback of requiring the incorporation of cysteine via genetic modification or the reduction of disulfide bonds in the antibody. Finally, the method utilizing an enzyme enables site-specific and efficient conjugation reactions; however, in most cases, it has the drawback of requiring genetic modification to incorporate amino acid sequences recognizable by the enzyme into the antibody.

Therefore, there is a need for the development of a method that enables the synthesis of site-selective and homogeneous ADCs while allowing the use of existing antibody production systems without genetic modification.

### [Related Art Document]

(Patent Document) Korean Patent Publication No. 10-2014-0046302.

### [Disclosure]

### [Technical Problem]

The present disclosure provides a conjugation-mediating protein comprising a binding protein into which non-canonical amino acids are incorporated and a pyridinium oxime derivative conjugated to the binding protein, a method for labeling an antibody using the conjugation-mediating protein, and a method for the site-specific synthesis of antibody-drug conjugates.

However, the problems to be solved by the present disclosure are not limited to the above-mentioned problems, and other problems not mentioned will be clearly understood by those skilled in the art from the following description.

### [Technical Solution]

In a first aspect, the present disclosure provides a conjugation-mediating protein comprising a binding protein into which non-canonical amino acids are incorporated and a pyridinium oxime derivative conjugated to the binding protein.

In a second aspect, the present disclosure provides a method for labeling an antibody using the conjugation-mediating protein.

In a third aspect, the present disclosure provides a method for the site-specific synthesis of antibody-drug conjugates using the conjugation-mediating protein.

### [Advantageous Effects]

According to the present disclosure, there is provided a method for synthesizing antibody-drug conjugates by site-selectively incorporating a drug into an antibody using a pyridinium oxime derivative (conjugation-mediating protein) incorporated into a binding protein containing a non-canonical amino acid.

The method for synthesizing antibody-drug conjugates according to the present disclosure is applicable to native antibodies, and is expected to have high utility because it does not require any additional modifications to existing antibody production systems.

Further, the method for synthesizing antibody-drug conjugates according to the present disclosure can proceed with a very high yield and ensures homogeneity. Thus, it is expected to be applicable to novel antibody drugs and various novel protein-based drugs.

### [Description of Drawings]

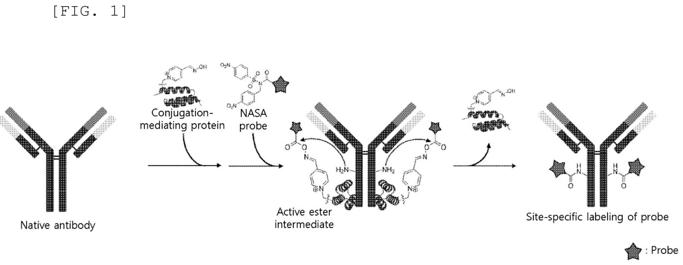

FIG. 1 shows a schematic diagram of a method for the site-specific synthesis of the antibody-drug conjugate, according to an embodiment of the present disclosure.

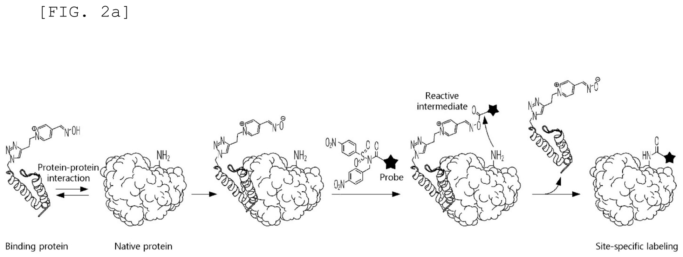

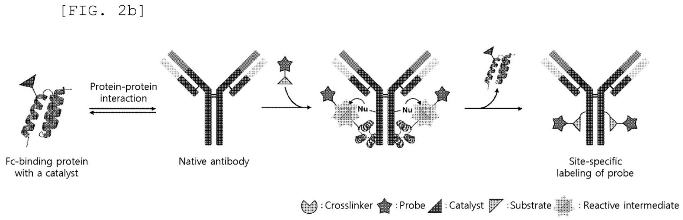

FIGS. 2a and 2b show schematic diagrams of an antibody labeling method, according to an embodiment of the present disclosure.
FIG. 3 shows a schematic diagram showing a protein composed of a Z-domain protein (Z-DM) and a Z-domain-specific affibody (Z-AFB), according to an embodiment of the present disclosure.
FIG. 4 shows the formulas of 4-azido-L-phenylalanine (AzF), N₆-[(2-azidoethoxy)carbonyl]-L-lysine (AzK), pyridinium oxime-alkyne (PyOx-Alkyne), PyOx_{_}C₆-Alkyne, N-alkylsulfonamide-fluorescein (NASA-FL), NASA-biotin (NASA-Bt), and NASA-N₃, according to an embodiment of the present disclosure.
FIG. 5 shows a schematic diagram illustrating a method for labeling Z-DM using a genetic code expansion method and Cu-catalyzed azide-alkyne cycloaddition (CuAAC), according to an embodiment of the present disclosure.
FIG. 6 shows a schematic diagram illustrating the position of Z-AFB into which non-canonical amino acids (ncAAs) are incorporated and the distance from the Z-AFB to the Z-DM labeling position, according to an embodiment of the present disclosure.
FIG. 7 shows MALDI-TOF analysis spectra performed using Z-DM, Z-AFB_K4AzF-PyOx, Z-AFB_K28AzF-PyOx, Z-AFB_F32AzF-PyOx, and Z-AFB_K28AzK-PyOx, according to an embodiment of the present disclosure.
FIG. 8 shows a graph illustrating the labeling efficiency according to Z-AFB_K4AzF-PyOx, Z-AFB_K28AzF-PyOx, Z-AFB_F32AzF-PyOx, and Z-AFB_K28AzK-PyOx, and a table showing the distances from K4, K28, and F32 in affibodies to the target lysine (Lys), according to an embodiment of the present disclosure.
FIG. 9 shows protease (Glu-C) digestion analysis spectra of a native Z-DM peptide fragment (peptide fragment 1) and FL-labeled Z-DM fragment (peptide fragment 2), according to an embodiment of the present disclosure.
FIG. 10 shows a MALDI-TOF/TOF tandem MS analysis spectrum of peptide fragment 1 (KGSVDNKFNKE, SEQ ID NO: 4), according to an embodiment of the present disclosure.
FIG. 11 shows a MALDI-TOF/TOF tandem MS analysis spectrum of peptide fragment 2 demonstrating FL labeling at K18, according to an embodiment of the present disclosure.
FIG. 12a shows a graph illustrating the labeling efficiency of Z-DM over time, according to an embodiment of the present disclosure, and FIG. 12b shows MALDI-TOF MS analysis spectra for Z-DM labeling at labeling times of 1, 2, 3, and 4 hours, according to an embodiment of the present disclosure.
FIGS. 13a and 13b show a schematic diagram illustrating labeling evaluations on non-binding proteins; and a graph illustrating labeling efficiency of strep-Z domain, Fc fragment, lysozyme, and TrpR, respectively, according to an embodiment of the present disclosure.
FIG. 14 shows a schematic diagram illustrating a method for labeling the Fc fragment of human immunoglobulin G1 (IgG1), according to an embodiment of the present disclosure.
FIG. 15 shows an overlaid image of the crystal structures of a Z-domain variant (Z-M) protein modeled using an AlphaFold2 program and a three-helix Fc-binding protein derived from PDB ID 5U4Y, according to an embodiment of the present disclosure.
FIG. 16 shows a schematic diagram illustrating the distance between the Z-M protein and the Fc fragment for optimal site selection of ncAA and PyOx incorporation in the Z-M protein, according to an embodiment of the present disclosure.
FIG. 17 shows an HPLC analysis graph of labeling experiments for Fc fragments using Z-M variants (Z-M_M8AzK-PyOx, Z-M_M8AzK-PyOx_C₆, Z-M_D37AzK-PyOx, Z-M_K33AzK-PyOx, and Z-M_H19AzK-PyOx) and NASA probes (NASA-FL and NASA-Bt); Fc fragments using NASA-FL; the Fc fragments, according to an embodiment of the present disclosure.
FIG. 18 shows a schematic diagram illustrating steric hindrance to the target lysines by H19, K33, and D37 of the Z-M protein, according to an embodiment of the present disclosure.
FIG. 19 shows a schematic diagram illustrating the distance between M8 of the Z-M protein and K248 and K246 of the Fc fragment, according to an embodiment of the present disclosure.
FIG. 20 shows protease (trypsin) digestion analysis spectra (MALDI-TOF/TOF tandem MS) of a native Fc fragment (peptide fragment 5), a FL-labeled Fc fragment (peptide fragment 6), and a Bt-labeled Fc fragment (peptide fragment 7), according to an embodiment of the present disclosure.
FIG. 21 shows a MALDI-TOF/TOF tandem MS analysis spectrum of peptide fragment 5 (ASDKTHTCPPCPAPELLGGPSVFLFPPKPKDTLMISR, SEQ ID NO: 6), according to an embodiment of the present disclosure.
FIG. 22 shows a MALDI-TOF/TOF tandem MS analysis spectrum of peptide fragment 6 demonstrating biotin labeling at K248, according to an embodiment of the present disclosure.
FIG. 23 shows a MALDI-TOF/TOF tandem MS analysis spectrum of peptide fragment 7 demonstrating FL labeling at K248, according to an embodiment of the present disclosure.
FIG. 24 shows an HPLC analysis graph of trastuzumab wild-type (WT) and Bt-labeled trastuzumab, according to an embodiment of the present disclosure.
FIG. 25 shows protease (trypsin) digestion analysis spectra (MALDI-TOF/TOF tandem MS) of a native trastuzumab fragment (peptide fragment 8) and a Bt-labeled trastuzumab fragment (peptide fragment 9), according to an embodiment of the present disclosure.
FIG. 26 shows a MALDI-TOF/TOF tandem MS analysis spectrum of peptide fragment 8 (GPSVFLFPPKPKDTLMISR, SEQ ID NO: 8), according to an embodiment of the present disclosure.
FIG. 27 shows a MALDI-TOF/TOF tandem MS analysis spectrum of peptide fragment 9, according to an embodiment of the present disclosure.
FIG. 28 shows a schematic diagram illustrating a labeling method for incorporating BCN-VC-PABC-MMAE onto trastuzumab using NASA-N₃, according to an embodiment of the present disclosure.
FIG. 29 shows HPLC analysis graphs of trastuzumab WT, trastuzumab-N₃, and trastuzumab-(VC-PABC-MMAE)₂, according to an embodiment of the present disclosure.
FIG. 30 shows HPLC analysis graphs of trastuzumab WT and trastuzumab-N₃, according to an embodiment of the present disclosure.
FIG. 31 shows Orbitrap-MS analysis graphs of trastuzumab-WT digested with IdeS, according to an embodiment of the present disclosure.
FIG. 32 shows Orbitrap-MS analysis graphs of trastuzumab-N₃ digested with IdeS, according to an embodiment of the present disclosure.
FIG. 33 shows protease (trypsin) digestion analysis spectra (MALDI-TOF/TOF tandem MS) of a native trastuzumab fragment (peptide fragment 10) and a trastuzumab-N₃ fragment (peptide fragment 11), according to an embodiment of the present disclosure.
FIG. 34 shows a MALDI-TOF/TOF tandem MS analysis spectrum of peptide fragment 10 (GPSVFLFPPKPKDTLMISR, SEQ ID NO: 8), according to an embodiment of the present disclosure.
FIG. 35 shows a MALDI-TOF/TOF tandem MS analysis spectrum of peptide fragment 11, according to an embodiment of the present disclosure.

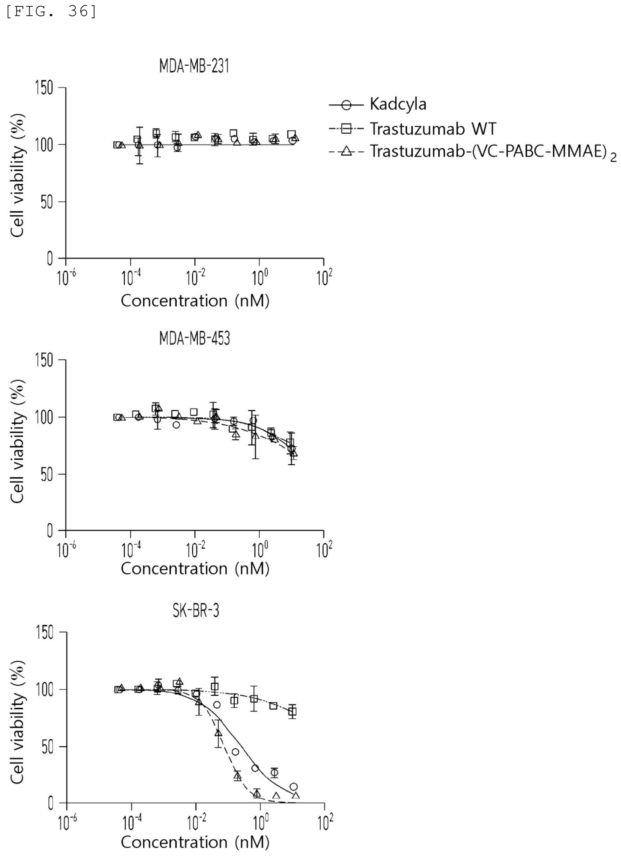

FIG. 36 shows graphs illustrating the *in vitro* antitumor activity of Kadcyla, trastuzumab WT, and trastuzumab-(VC-PABC-MMAE)₂ on MDA-MB-231, MDA-MB-453, and SK-BR-3 at various concentrations, according to an embodiment of the present disclosure.

### [Best Mode]

Hereinafter, embodiments and examples of the present disclosure will be described in detail with reference to the accompanying drawings to enable those of ordinary skill in the art to which the present disclosure pertains to readily practice the present disclosure. However, the present disclosure may be embodied in various different forms and is not limited to the embodiments and examples described herein. In the drawings, portions unrelated to the description have been omitted to clearly illustrate the present disclosure, and similar reference numerals have been assigned to similar parts throughout the specification.

Throughout the present specification, when a part is described as being "connected" to another part, it includes not only the case of being "directly connected," but also the case of being "electrically connected" with other elements interposed therebetween.

Throughout the present specification, when a member is described as being "on" another member, it includes not only the case where the member is in direct contact with the other member, but also the case where another member is interposed therebetween.

Throughout the present specification, "comprising" any component will be understood to imply the inclusion of other components rather than the exclusion of other components, unless otherwise specifically stated.

As used herein, the terms such as "about", "substantially," etc., are used to mean at or near the numerical value when manufacturing and material tolerances inherent in the stated meanings are present, and are used to prevent an unscrupulous infringer from unfairly taking advantage of a disclosure that mentions a precise or absolute numerical value, to assist in the understanding of the present disclosure.

Throughout the present specification, the terms "step of ~ing" or "step of ~" are not intended to mean "step for ~."

Throughout the present specification, the term "combination(s) thereof" included in a Markush-type expression refers to a mixture of one or more components or combinations thereof selected from the group consisting of the components described in the Markush-type expression, and is intended to include one or more selected from the group consisting of the aforementioned components.

Throughout the present specification, the expression "A and/or B" refers to "A or B, or A and B."

Throughout the present specification, when functional groups are substituted, they may include, but are not limited to, one or more selected from an acyl group, an amino group (including a simple amino group, a mono- and dialkylamino group, a mono- and diarylamino group, and an alkylaryl amino group), an acylamino group (including a carbamoyl group and an ureido group), an alkylcarbonyloxy group, an arylcarbonyloxy group, an alkoxycarbonyloxy group, an alkoxycarbonyl group, a carboxyl group, a carboxylate group, an aminocarbonyl group, a mono- and dialkylaminocarbonyl group, a cyano group, an azido group, a halogen group, a hydroxyl group, a nitro group, a trifluoromethyl group, a thiol group, an alkylthiol group, an arylthiol group, an alkylthiocarbonyl group, a thiocarboxylate group, an alkyl group (including, but not limited to, having 1 to 20 carbon atoms); an alkenyl group (including, but not limited to, having 2 to 20 carbon atoms), an alkynyl group (including, but not limited to, having 2 to 20 carbon atoms), a cycloalkyl group (including, but not limited to, having 3 to 20 carbon atoms), a heterocycloalkyl group (including, but not limited to, having 3 to 20 carbon atoms), an aryl group (including, but not limited to, having 4 to 20 carbon atoms), a heteroaryl group (including, but not limited to, having 3 to 20 carbon atoms), a lower alkoxy group, an aryloxy group, an aryloxycarbonyloxy group, a benzyloxy group, a benzyl group, a sulfinyl group, an alkylsulfinyl group, a sulfonyl group, a sulfate group, a sulfonate group, a sulfonamide group, a phosphate group, a phosphonato group, a phosphinato group, an oxo group, a guanidine group, an imino group, and a formyl group.

Hereinafter, embodiments of the present disclosure are described in detail; however, the present disclosure is not limited thereto.

In a first aspect, the present disclosure provides a conjugation-mediating protein comprising a binding protein into which non-canonical amino acids are incorporated and a pyridinium oxime derivative conjugated to the binding protein.

As used herein, the "binding protein" refers to any type of protein into which a non-canonical amino acid as described below, may be genetically incorporated. Here, the "genetically incorporated" refers to substituting one or more amino acids in the binding protein with other amino acids by substituting the nucleic acid sequences corresponding to one or more amino acids in the binding protein with other nucleic acid sequences, regardless of the type of the binding protein.

For example, the binding protein may be a protein derived from protein A, protein G, protein L, or an analogous protein having the same function, all of which are known to bind to antibodies, but the present disclosure is not limited thereto.

Specifically, the binding protein may be a B-domain variant of Staphylococcal Protein A, which is present in *Staphylococcus aureus* cells.

According to an embodiment of the present disclosure, the B-domain variant comprises an amino acid sequence set forth in SEQ ID NO: 1 or SEQ ID NO: 2 (hereinafter also referred to as "Z-M").

As used herein, the "non-canonical amino acids" refer to any amino acids other than the 20 common amino acids, selenocysteine, and pyrrolysine. Other terms that may be used in a similar sense to "non-canonical amino acids" include "unnatural amino acids," "non-naturally encoded amino acids," and "amino acids not occurring in nature," but their scientific definitions may vary slightly. The naturally encoded amino acids include, but is not limited to, the 20 common amino acids, selenocysteine, or pyrrolysine.

For example, the non-canonical amino acid may be selected from the group consisting of, but is not limited to, 4-azido-L-phenylalanine, N₆-((2-azidoethoxy)carbonyl)-L-lysine, 4-acetyl-phenylalanine, O-propargyl-tyrosine, N₆-(propargyloxy)carbonyl-lysine, N₆-(bicyclononyloxy)carbonyl-lysine, N₆-(((2-methylcyclopropenyl)methoxy)carbonyl)-lysine, N₆-(trans-cyclooctenyloxy)carbonyl-lysine, and 4-(6-methyltetrazinyl)phenylalanine, which are amino acids represented by the following Formulas 1 to 9:

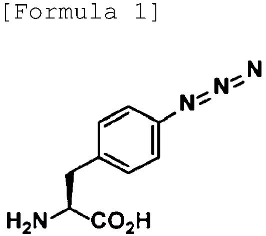

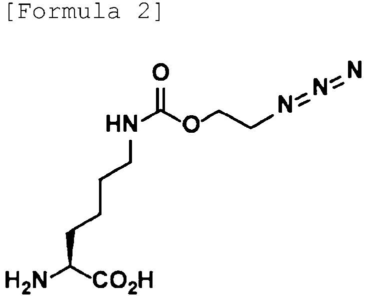

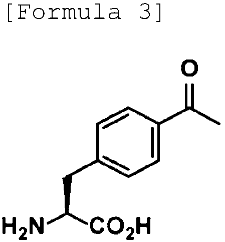

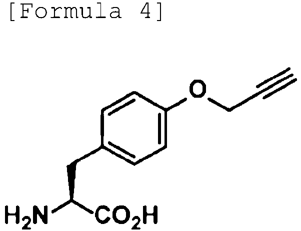

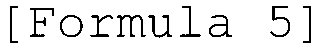

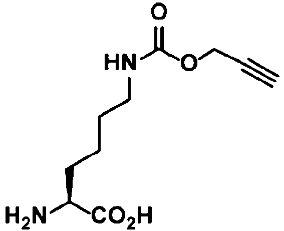

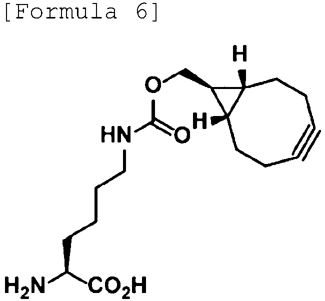

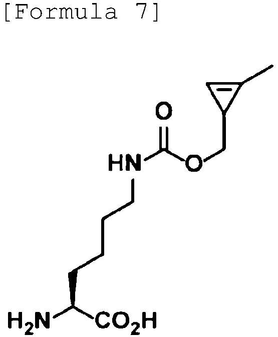

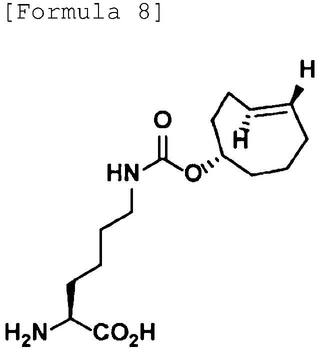

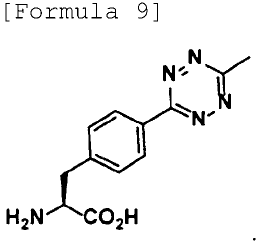

Specifically, the non-canonical amino acid may be 4-azido-L-phenylalanine, N₆-((2-azidoethoxy)carbonyl)-L-lysine, O-propargyl-tyrosine, or N₆-(propargyloxy)carbonyl-lysine.

According to an embodiment of the present disclosure, the non-canonical amino acid may be incorporated by substituting one or more amino acids included in the binding protein.

The positions of the amino acid in the binding protein that may be substituted with the non-canonical amino acid include, but are not limited to, positions excluded from potential receptor-binding sites that bind to one or more binding partners, positions that may be fully or partially exposed to the solvent, positions having minimal or no hydrogen bonding interaction with adjacent residues, positions minimally exposed to adjacent reactive residues, positions that, based on the predicted three-dimensional structure of the protein, are highly flexible or structurally rigid, positions in a site bound or unbound to a related receptor, ligand, or binding protein, positions in a site bound or unbound to another protein or other biologically active molecule, or positions capable of modulating the conformation of a protein itself, or a dimer or multimer comprising one or more proteins by altering the flexibility or rigidity of the overall structure as desired.

According to an embodiment of the present disclosure, based on the X-ray crystal structures of the Z domain and affibody, positions near the binding site were identified without affecting their mutual binding, and the positions (e.g., K7 or D36) were selected for incorporating a non-canonical amino acid.

Furthermore, according to another embodiment of the present disclosure, based on the crystal structure of a complex formed by a two-helix Fc-binding protein and an Fc fragment, positions near the Fc fragment in the resulting three-helix Fc-binding protein (Z-M) were identified while minimizing disruption of Fc binding, and the positions (e.g., M8, H19, K33, or D37) were selected for incorporating a non-canonical amino acid.

The binding protein into which non-canonical amino acids are incorporated, prepared according to the above embodiment, comprises the amino acid sequence set forth in SEQ ID NO: 3.

Specifically, the amino acid sequence set forth in SEQ ID NO: 3 is a sequence in which the methionine (M) at the 8th position of the Z-domain variant (Z-M), which is a binding protein, is substituted with a non-canonical amino acid, N₆-[(2-azidoethoxy)carbonyl]-L-lysine (AzK).

As used herein, the "pyridinium oxime derivative" may be represented by the following Formula 1, but the present disclosure is not limited thereto:

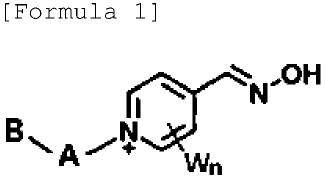

wherein:
A is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 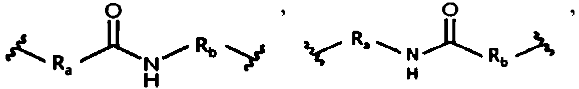 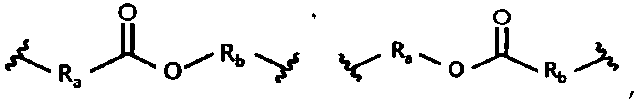 and combinations thereof,
Rₐ and R_{b} are each independently absent or a substituted or unsubstituted C₁-C₁₀ alkylene group, or a substituted or unsubstituted C₂-C₁₀ polyethylene glycol group,
B is selected from the group consisting 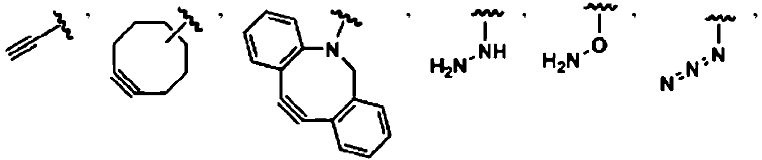 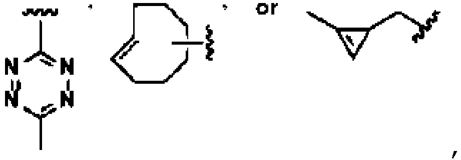
W is -F, -Cl, -Br, -NO₂, or -CF₃, and
n is an integer from 0 to 4.

Here, the C₁-C₁₀ alkylene group may be selected from the group consisting of a methylene group, an ethylene group, a propylene group, a butylene group, a pentylene group, a hexylene group, a heptylene group, a nonylene group, a decylene group, and isomers thereof.

Here, the C₂-C₁₀ polyethylene glycol may be represented by [-(C₂H₄)-O-]ₘ, where m may be an integer from 1 to 5.

Here, depending on the combination of linkers listed as A, the linking moiety in a form such as -(CH₂)ₒ-O-(CH₂)ₚ-, -(CH₂)ₒ-(C₆H₄)-(CH₂)ₚ-, -(CH₂)ₒ-NH-(CH₂)ₚ-, -(CH₂)ₒ-NH-(C₆H₄)-(CH₂)ₚ-, -(CH₂)ₒ-O-(C₆H₄)-(CH₂)ₚ-, -(CH₂)ₒ-(C₆H₄)-NH-(CH₂)ₚ-, or - (CH₂)ₒ-(C₆H₄)-O-(CH₂)ₚ- (wherein o and p are each independently an integer from 0 to 5) may also be present.

The hydroxyl group present in the pyridinium oxime has a pKₐ value of 8 or less, and exists in an anionic state under typical physiological conditions, thereby enabling an efficient and selective reaction with a labeling material (a binding protein into which non-canonical amino acids are incorporated).

According to an embodiment of the present disclosure, the non-canonical amino acids incorporated into the binding protein and the pyridinium oxime derivative may be subjected to bioorthogonal reactions, such as copper(I)-catalyzed azide-alkyne cycloaddition (CuAAC) or strain-promoted azide-alkyne cycloaddition (SPAAC), but the present disclosure is not limited thereto.

Specifically, the bioorthogonal reaction may be a reaction between B of the pyridinium oxime derivative of Formula 1 and a terminal functional group of the non-canonical amino acids described above.

For example, the structure formed according to the reaction in which B of the pyridinium oxime derivative of Formula 1 reacts with and binds to the terminal functional group of the non-canonical amino acid described above, may be as follows:

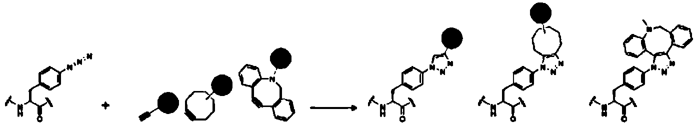

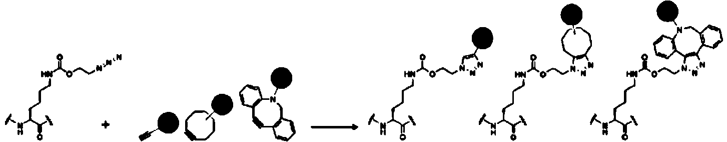

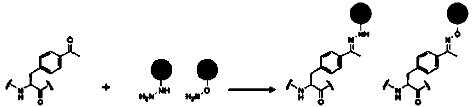

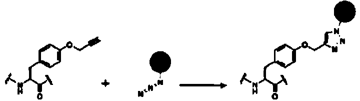

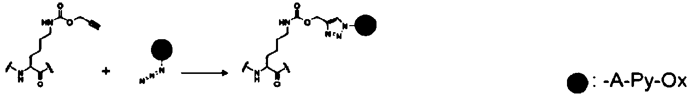

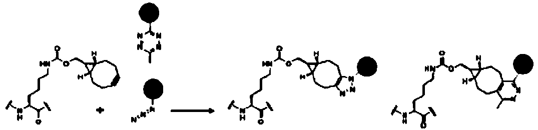

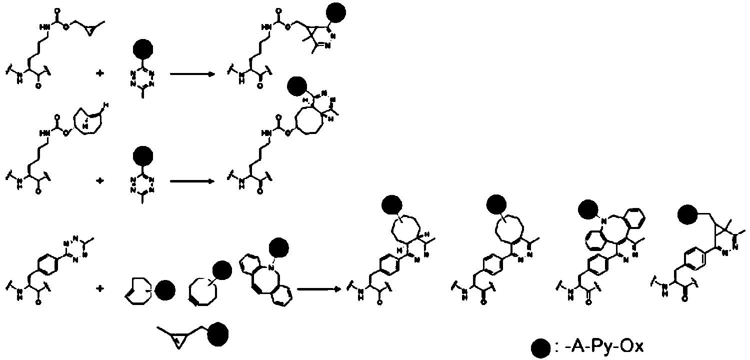

The CuAAC may proceed efficiently in the presence of copper ions (Cu²⁺), a reducing agent (ascorbic acid, which convers Cu²⁺ to Cu⁺), and a ligand (which stabilizes copper ion).

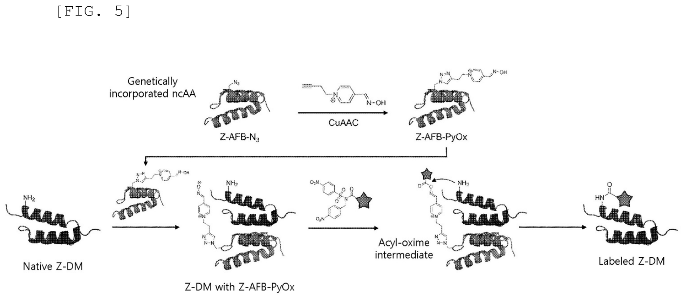

The CuAAC is a reaction having high sensitivity and specificity. FIG. 5 is a schematic diagram illustrating the process of CuAAC. As shown in FIG. 5, the azide group (N₃) contained in the binding protein into which azide group (N₃) -containing non-canonical amino acids are genetically incorporated, undergoes a conjugation reaction with a pyridinium oxime derivative via CuAAC.

The pyridinium oxime derivative and CuAAC are well known to be ideal for bioconjugation of biochemical molecules such as proteins, as they have high rate constants (0.1 to 1.0 M⁻¹·s⁻¹) and the triazole structure formed after the reaction is relatively small. To apply CuAAC to protein conjugation, the binding protein is required to have an azide group (alkyne group) incorporated. Since the incorporation of an azide group (alkyne group) into a binding protein may be achieved by genetic methods, applying CuAAC to genetically incorporated azide group (alkyne group)-containing amino acids may enable effective labeling of the binding protein.

The conjugation reaction may be initiated by mixing the binding protein and the pyridinium oxime derivative, but present disclosure is not limited thereto.

In the present disclosure, the nucleic acid sequence encoding the "binding protein into which non-canonical amino acids are incorporated" is based on the amino acid sequence of a wild-type protein, and the nucleic acid sequence corresponding to the amino acid sequence may be modified to introduce, substitute, or delete specific amino acids included in the amino acid sequence, but the present disclosure is not limited thereto. The nucleic acid sequence may be selectively or non-selectively modified according to methods known in the art. For example, the method may include, but is not limited to, replacing a codon encoding a specific amino acid in the gene of the protein with an amber codon (TAG); introducing a plasmid including a gene of the protein having the substituted stop codon, a plasmid including a suitable tRNA gene, and a suitable aminoacyl-tRNA synthetase gene into a cell, for example, *Escherichia coli,* to produce a transformed cell; and culturing the transformed cell in a medium containing the non-canonical amino acid.

In the production of the protein variant by the transformed cell, the non-canonical amino acids may be bound to the tRNA by the aminoacyl-tRNA synthetase, and the tRNA bound to the non-canonical amino acids may recognize the substituted nucleic acid sequence (TAG) to insert the non-canonical amino acids into an appropriate site of the translated protein, but the present disclosure is not limited thereto.

The non-canonical amino acids may be present at any position on the protein, including any terminal position or any internal position. The protein into which the non-canonical amino acids are incorporated may be produced biosynthetically or through solid-phase peptide synthesis, but the present disclosure is not limited thereto. The "biosynthetically" refers to a method using a cellular translation system, including, but not limited to, the use of one or more of a polynucleotide, a codon, tRNA, and a ribosome.

As used herein, the "conjugation-mediating protein" refers to a protein that does not directly include a labeling substance, but serves as a mediator that binds to a target protein to catalyze labeling.

In a second aspect, the present disclosure provides a method for labeling an antibody, comprising:
a first step of binding the conjugation-mediating protein described above to a native antibody;
a second step of reacting the conjugation-mediating protein with a probe-bound N-alkylsulfonamide derivative to form an active ester intermediate; and
a third step of reacting lysine contained in an Fc domain of the native antibody with the intermediate to form an amide bond.

According to an embodiment of the present disclosure, the first, second, and third steps in which the method for labeling an antibody is carried out are described sequentially. However, this order is provided merely to illustrate the reaction, and the order of each step is not limited. Specifically, the method for labeling an antibody may involve reactions that occur substantially simultaneously or nearly simultaneously.

According to an embodiment of the present disclosure, the first step may be performed by mixing a pyridinium oxime derivative-labeled binding protein (conjugation-mediating protein) with a native antibody.

As used herein, the "native antibody" refers to any type of antibody that naturally occurs as a result of the function of an intact human immune system, and may be used interchangeably with "natural antibody" or "complete antibody." The native antibody differs in some aspects from antibodies obtained by recombinant library methods (phage display or transgenic mice) and has unique properties that may make it an ideal therapeutic for human viral diseases.

A complete antibody has a structure comprising two full-length light chains and two full-length heavy chains, wherein each light chain is linked to a heavy chain via disulfide bonds. The heavy chain constant region includes gamma (γ), mu (µ), alpha (α), delta (δ), and epsilon (ε) types, with subclasses of gamma 1 (γ1), gamma 2 (γ2), gamma 3 (γ3), gamma 4 (γ4), alpha 1 (α1), and alpha 2 (α2). The light chain constant region has kappa and lambda types (Cellular and Molecular Immunology, Wonsiewicz, M. J., Ed., Chapter 45, pp. 41-50, W. B. Saunders Co., Philadelphia, PA (1991); Nisonoff, A., Introduction to Molecular Immunology, 2nd Ed., Chapter 4, pp. 45-65, Sinauer Associates, Inc., Sunderland, MA (1984)).

As used herein, the "heavy chain" refers to a full-length heavy chain comprising a variable domain VH which comprises an amino acid sequence having a sufficient variable region sequence to confer antigen specificity, and three constant domains CH1, CH2, and CH3, as well as fragments thereof. Also, as used herein, the "light chain" refers to a full-length light chain comprising a variable domain VL which comprises an amino acid sequence having a sufficient variable region sequence to confer antigen specificity and a constant domain CL, as well as fragments thereof.

Specifically, the native antibody may be human immunoglobulin G1 (IgG1), but present disclosure is not limited thereto.

According to an embodiment of the present disclosure, the second step may be performed by mixing the probe-bound N-alkylsulfonamide derivative with the native antibody bound to the conjugation-mediating protein.

As used herein, the "probe-bound N-alkylsulfonamide (NASA) derivative" refers to an N-alkylsulfonamide group or a derivative thereof substituted with a probe (P), which may be represented by the following Formula 2, but the present disclosure is not limited thereto:

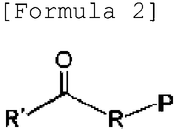

wherein:
R' is 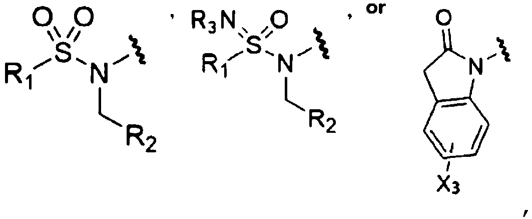
R is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 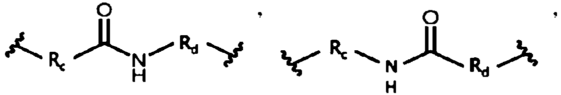 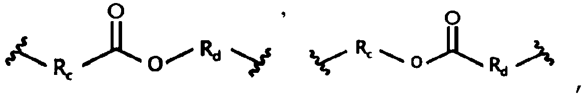 and combinations thereof,
R_{c} and R_{d} are each independently absent or a substituted or unsubstituted C₁-C₁₀ alkylene group, or a substituted or unsubstituted C₂-C₁₀ polyethylene glycol group,
R₁ is selected from CF₃ or 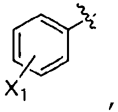
R₂ is selected from CF₃, 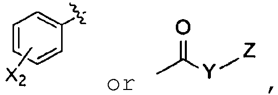
R₃ is H or a linear or branched C₁-C₅ alkyl group,
P is selected from 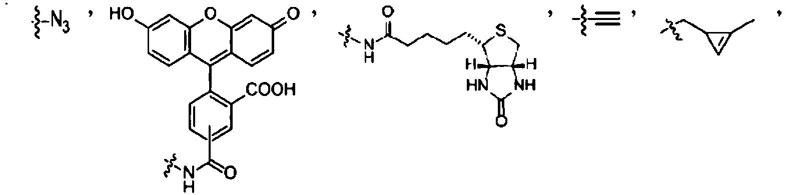 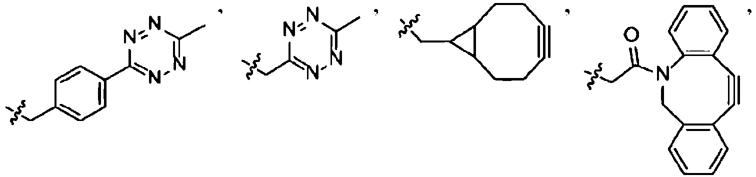 or 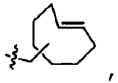
X₁, X₂, and X₃ substitute one or more hydrogen atoms on the phenyl group, and are each independently F, Cl, Br, CF₃, or NO₂,
Y is O or NH, and
Z is a linear or branched C₁-C₂₀ alkyl group which is unsubstituted or substituted with -NRₑR_{f}, wherein
Rₑ and R_{f} are each independently a C₁-C₅ alkyl group, or a C₄-C₁₀ cyclic alkyl group linked to each other, wherein the ring may optionally contain heteroatoms selected from N, S, or O.

Here, the C₁-C₁₀ alkylene group may be selected from the group consisting of a methylene group, an ethylene group, a propylene group, a butylene group, a pentylene group, a hexylene group, a heptylene group, a nonylene group, a decylene group, and isomers thereof.

Here, the C₂-C₁₀ polyethylene glycol group may be represented as [-(C₂H₄)-O-]ₘ, where m may be an integer from 1 to 5.

Here, depending on the combination of the C₁-C₁₀ alkylene group and the C₂-C₁₀ polyethylene glycol group, the linking moiety in a form such as -(CH₂)-(CH₂CH₂)-O- or - (CH₂CH₂)-(CH₂CH₂)-O- may also be present.

Here, depending on the combination of linkers listed as R, the linking moiety in a form such as -(CH₂)ₒ-O-(CH₂)ₚ-, -(CH₂)ₒ-(C₆H₄)-(CH₂)ₚ-, -(CH₂)ₒ-NH-(CH₂)ₚ-, -(CH₂)ₒ-NH-(C₆H₄)-(CH₂)ₚ-, -(CH₂)ₒ-O-(C₆H₄)-(CH₂)ₚ-, -(CH₂)ₒ-(C₆H₄)-NH-(CH₂)ₚ-, or -(CH₂)ₒ-(C₆H₄)-O-(CH₂)ₚ- (wherein o and p are each independently an integer from 0 to 5) may also be present.

Specifically, the probe-bound *N*-alkylsulfonamide derivative may be *N*-alkylsulfonamide-fluorescein (NASA-FL), *N*-alkylsulfonamide-biotin (NASA-Bt), *N*-alkylsulfonamide-N₃, *N*-alkylsulfonamide-thiol, tetrazine, cyclopropene, trans-cyclooctene, bicyclo[6.1.0]non-4-yne (BCN), and/or dibenzocyclooctyne (DBCO).

More specifically, the probe-bound *N-*alkylsulfonamide derivative may be *N*-alkylsulfonamide-fluorescein (NASA-FL), *N*-alkylsulfonamide-biotin (NASA-Bt), or *N*-alkylsulfonamide-N₃.

As used herein, the conjugation-mediating protein and the probe-bound *N*-alkylsulfonamide derivative may specifically react with each other to form an active ester intermediate.

As used herein, the "active ester intermediate" refers to an acyl-oxime intermediate.

As used herein, the active ester intermediate may be bound to lysine contained in the Fc domain of the native antibody.

Specifically, the active ester intermediate and lysine may be bound by forming an amide bond.

As used herein, the "Fc domain" refers to a single heavy chain Fc region of an antibody, and may comprise one to four domains selected from the group consisting of CH1, CH2, CH3, and CH4 domains.

Specifically, the Fc domain may further include a hinge region.

More specifically, the Fc domain may be selected from the group consisting of IgG, IgA, IgD, IgE, IgM, combinations thereof, and hybrids thereof.

According to an embodiment of the present disclosure, the Fc domain protein comprises the amino acid sequence set forth in SEQ ID NO: 5.

Detailed descriptions of portions overlapping with the first aspect of the present application have been omitted; however, the descriptions of the first aspect of the present disclosure may be applied equally to the second aspect of the present disclosure, even if the descriptions are omitted.

In a third aspect, the present disclosure provides a method for the site-specific synthesis of antibody-drug conjugates, comprising:
a first step of binding the conjugation-mediating protein described above to a native antibody;
a second step of reacting the conjugation-mediating protein with a probe-bound *N*-alkylsulfonamide derivative to form an active ester intermediate;
a third step of reacting lysine contained in an Fc domain of the native antibody with the intermediate to form an amide bond; and
a fourth step of reacting the probe bound to the lysine with a drug to label the drug,
alternatively,
a first step of binding the conjugation-mediating protein described above to a native antibody;
a second step of reacting the conjugation-mediating protein with a drug-bound *N*-alkylsulfonamide derivative to form an active ester intermediate; and
a third step of reacting lysine contained in an Fc domain of the native antibody with the intermediate to form an amide bond.

As used herein, the "probe-bound *N-*alkylsulfonamide (NASA) derivative" refers to an N-alkylsulfonamide group or a derivative thereof substituted with a probe (P), which may be represented by the following Formula 2, but the present disclosure is not limited thereto:

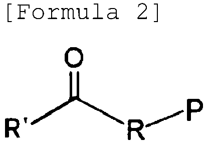

wherein:
R' is 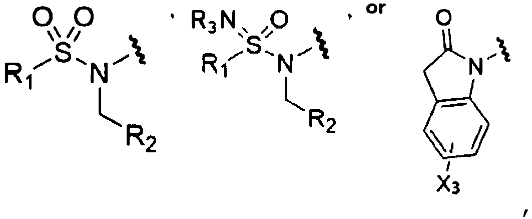
R is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 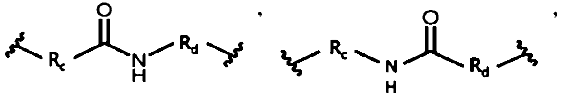 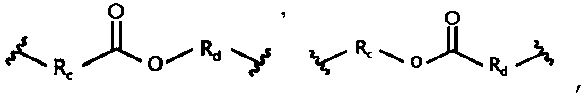 and combinations thereof,
R_{c} and R_{d} are each independently absent or a substituted or unsubstituted C₁-C₁₀ alkylene group, or a substituted or unsubstituted C₂-C₁₀ polyethylene glycol group,
R₁ is selected from CF₃ or 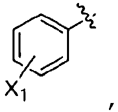
R₂ is selected from CF₃, 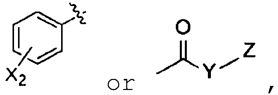
R₃ is H or a linear or branched C₁-C₅ alkyl group,
P is selected from 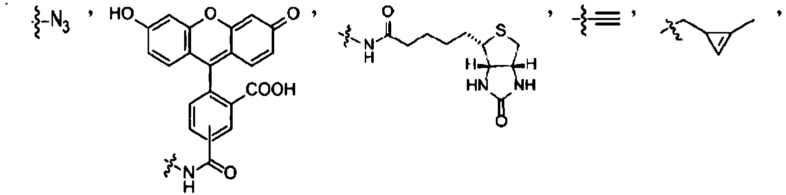 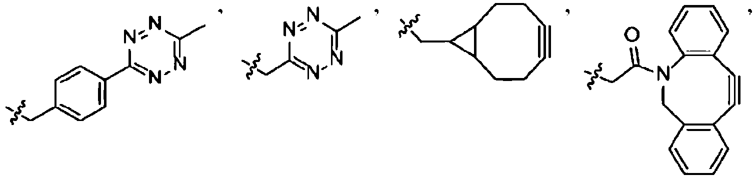 or 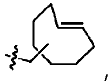
X₁, X₂, and X₃ substitute one or more hydrogen atoms on the phenyl group, and are each independently F, Cl, Br, CF₃, or NO₂,
Y is O or NH, and
Z is a linear or branched C₁-C₂₀ alkyl group which is unsubstituted or substituted with -NRₑR_{f}, wherein
Rₑ and R_{f} are each independently a C₁-C₅ alkyl group, or a C₄-C₁₀ cyclic alkyl group linked to each other, wherein the ring may optionally contain heteroatoms selected from N, S, or O.

Here, the C₁-C₁₀ alkylene group may be selected from the group consisting of a methylene group, an ethylene group, a propylene group, a butylene group, a pentylene group, a hexylene group, a heptylene group, a nonylene group, a decylene group, and isomers thereof.

Here, the C₂-C₁₀ polyethylene glycol group may be represented as [-(C₂H₄)-O-]ₘ, where m may be an integer from 1 to 5.

Here, depending on the combination of the C₁-C₁₀ alkylene group and the C₂-C₁₀ polyethylene glycol group, the linking moiety in a form such as -(CH₂)-(CH₂CH₂)-O- or - (CH₂CH₂)-(CH₂CH₂)-O- may also be present.

Here, depending on the combination of linkers listed as R, the linking moiety in a form such as -(CH₂)ₒ-O-(CH₂)ₚ-, -(CH₂)ₒ-(C₆H₄)-(CH₂)ₚ-, -(CH₂)ₒ-NH-(CH₂)ₚ-, -(CH₂)ₒ-NH-(C₆H₄)-(CH₂)ₚ-, -(CH₂)ₒ-O-(C₆H₄)-(CH₂)ₚ-, -(CH₂)ₒ-(C₆H₄)-NH-(CH₂)ₚ-, or -(CH₂)ₒ-(C₆H₄)-O-(CH₂)ₚ- (wherein o and p are each independently an integer from 0 to 5) may also be present.

Specifically, the probe-bound *N*-alkylsulfonamide derivative may be *N*-alkylsulfonamide-fluorescein (NASA-FL), *N*-alkylsulfonamide-biotin (NASA-Bt), *N*-alkylsulfonamide-N₃, *N*-alkylsulfonamide-thiol, tetrazine, cyclopropene, trans-cyclooctene, bicyclo[6.1.0]non-4-yne (BCN), and/or dibenzocyclooctyne (DBCO).

More specifically, the probe-bound *N-*alkylsulfonamide derivative may be *N*-alkylsulfonamide-fluorescein (NASA-FL), *N*-alkylsulfonamide-biotin (NASA-Bt), or *N*-alkylsulfonamide-N₃.

As used herein, the conjugation-mediating protein and the probe-bound *N*-alkylsulfonamide derivative may specifically react with each other to form an active ester intermediate.

As used herein, the "probe" bound to the lysine may be a moiety represented as P in the probe-bound *N-*alkylsulfonamide derivative represented by Formula 2.

In an embodiment of the present disclosure, when the conjugation-mediating protein directly reacts with a drug-bound *N*-alkylsulfonamide derivative, the drug may function as a probe. In this case, the drug-bound *N-*alkylsulfonamide derivative may be represented by the following Formula 3:

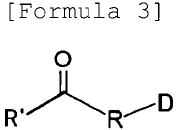

wherein:
R' is 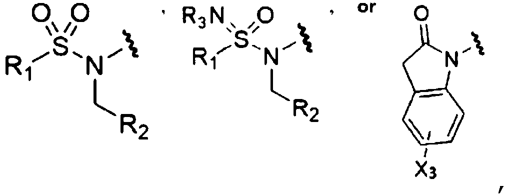
R is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 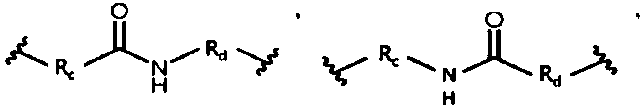 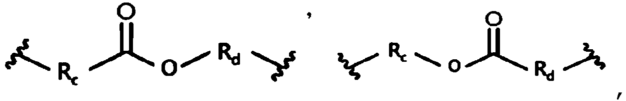 and combinations thereof,
R_{c} and R_{d} are each independently absent or a substituted or unsubstituted C₁-C₁₀ alkylene group, or a substituted or unsubstituted C₂-C₁₀ polyethylene glycol group,
R₁ is selected from CF₃ or 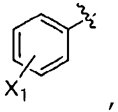
R₂ is selected from CF₃, 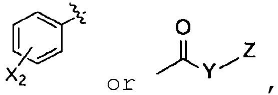
R₃ is H or a linear or branched C₁-C₅ alkyl group,
D is a drug or a drug comprising a cleavable linker,
X₁, X₂, and X₃ substitute one or more hydrogen atoms on the phenyl group, and are each independently F, Cl, Br, CF₃, or NO₂,
Y is O or NH, and
Z is a linear or branched C₁-C₂₀ alkyl group which is unsubstituted or substituted with -NRₑR_{f}, wherein
Rₑ and R_{f} are each independently a C₁-C₅ alkyl group, or a C₄-C₁₀ cyclic alkyl group linked to each other, wherein the ring may optionally contain heteroatoms selected from N, S, or O.

Here, the C₁-C₁₀ alkylene group may be selected from the group consisting of a methylene group, an ethylene group, a propylene group, a butylene group, a pentylene group, a hexylene group, a heptylene group, a nonylene group, a decylene group, and isomers thereof.

Here, the C₂-C₁₀ polyethylene glycol group may be represented as [-(C₂H₄)-O-]ₘ, where m may be an integer from 1 to 5.

Here, depending on the combination of the C₁-C₁₀ alkylene group and the C₂-C₁₀ polyethylene glycol group, the linking moiety in a form such as -(CH₂)-(CH₂CH₂)-O- or - (CH₂CH₂)-(CH₂CH₂)-O- may also be present.

Here, depending on the combination of linkers listed as R, the linking moiety in a form such as -(CH₂)ₒ-O-(CH₂)ₚ-, -(CH₂)ₒ-(C₆H₄)-(CH₂)ₚ-, -(CH₂)ₒ-NH-(CH₂)ₚ-, -(CH₂)ₒ-NH-(C₆H₄)-(CH₂)ₚ-, -(CH₂)ₒ-O-(C₆H₄)-(CH₂)ₚ-, -(CH₂)ₒ-(C₆H₄)-NH-(CH₂)ₚ-, or -(CH₂)ₒ-(C₆H₄)-O-(CH₂)ₚ- (wherein o and p are each independently an integer from 0 to 5) may also be present.

As used herein, the "drug (payload)" refers to a low molecular weight synthetic compound that accounts for a significant portion of the actual anticancer effect, and is one of the components of an antibody-drug conjugate that is as important as the antibody. In the present disclosure, the drug includes any type of compound that exhibits cytotoxic and anticancer effects.

According to an embodiment of the present disclosure, the drug may be a drug or a drug comprising a cleavable linker.

The method for the site-selective synthesis of antibody-drug conjugates according to an embodiment of the present disclosure, may be applied to any drug applicable to antibody-drug conjugates without limitation, unless otherwise limited in the application of the present disclosure. For example, the drug may be selected from the group consisting of monomethyl auristatin E (MMAE), Maytansinoids (including, but not limited to, DM1 and DM4), Calicheamicin, exatecan, SN-38, monomethyl auristatin F (MMAF), and derivatives thereof.

In an embodiment of the present disclosure, the linker may, without limitation, be any linker commonly used in the art and useful for antibody-drug conjugates. Here, the linker serves as a chemical linker that attaches the antibody to the drug, and is required to remain stable during circulation or distribution *in vivo,* but to be selectively cleaved to release the bound drug only when it penetrates the tumor and enters the cell. The linker typically comprises an attachment-spacer-release structure, and new linkers may be developed by modifying and/or improving one or more of the attachment, spacer, and release components of commercially available linkers. In an embodiment of the present disclosure, the linker may be, without limitation, not only commercially available linkers in the art but also newly developed linkers.

In an embodiment of the present disclosure, the linker may be a "cleavable linker," including, but not limited to, a disulfide linker such as N-succinimidyl-4-(2-pyridyldithio)pentanoate (SPP), N-succinimidyl-4-(2-pyridyldithio)butanoate (SDPB), sulfo-SPDB, MDS, DMDS, DSDM, or NDMDS; an enzymatically cleavable linker such as Phe-Lys-PABC, Val-Cit-PABC, Val-Ala-PABC, MHVCBC (valine-citrulline), or MHFKBC (phenylalanine-lysine); a hydrazone linker such as MHH; or a glucosidase-sensitive linker such as a glucuronic acid (GBC) linker, a glucuronic acid (GBCDN) linker, or a β-glucuronide linker; or a "non-cleavable linker," including, but not limited to, Mal-PEG-NHS, N-succinimidyl 4-(maleimidomethyl)cyclohexanecarboxylate (SMCC), a Mal-alkane linker, bis-maleimidopolyethyleneglycol (BMPEO), N-(β-maleimidopropyloxy)succinimide ester (BMPS), ε-maleimidocaproic acid N-hydroxysuccinimide ester (EMCS), γ-maleimidobutyric acid N-succinimidyl ester (GMBS), m-maleimidobenzoyl-N-hydroxysuccinimide ester (MBS), 4-(4-N-maleimidophenyl)-butyric acid hydrazide (MPBH), N-succinimidyl 3-(bromoacetamido)propionate (SBAP), N-succinimidyl iodoacetate (SIA), N-succinimidyl-4-(iodoacetyl)-aminobenzoate (SIAB), N-succinimidyl 4-(p-maleimidophenyl)butyrate (SMPB), or succinimidyl-6-(maleimidopropionamido)hexanoate (SMPH).

Specifically, the "cleavable linker" may be Val-Cit-p-aminobenzyloxycarbonyl (Val-Cit-PABC), but present disclosure is not limited thereto.

The drug may be incorporated in a form in which the probe is bound to the native antibody and then the drug is bound to the probe, or may be incorporated into the native antibody by using the drug-bound *N*-alkylsulfonamide derivative described above, thereby forming an amide bond with the antibody via a conjugation-mediating protein.

Detailed descriptions of portions overlapping with the first and/or second aspects of the present disclosure has been omitted, but the descriptions of the first and/or second aspects of the present disclosure may be equally applicable to the third aspect of the present disclosure, even if the descriptions have been omitted.

Hereinafter, the present disclosure will be described in more detail with reference to Examples. However, the following examples are provided merely to facilitate understanding of the present disclosure and the contents of the present disclosure are not limited to the following examples.

### [Examples]

### 1. Experimental Materials and Analytical Methods

### 1.1. Experimental Materials

All materials required for protein labeling were purchased and used without further purification.

For the incorporation of non-canonical amino acids (ncAAs), N₆-[(2-azidoethoxy)carbonyl]-L-lysine (AzK) was purchased from Iris Biotech, and 4-azido-L-phenylalanine (AzF) was purchased from Santa Cruz Biotechnology. NaCl, Na₂HPO₄, KH₂PO₄, (NH₄)₂SO₄, MgSO₄, CuSO₄, NaOH, ethylenediaminetetraacetic acid (EDTA), glycerol, sodium-L-ascorbate, aminoguanidine hydrochloride, and amino acids were purchased from Samchun Chemicals. Lactose and sodium dodecyl sulfate (SDS) were purchased from Sigma-Aldrich. Arabinose and chloramphenicol were purchased from Alfa Aesar. Ampicillin, kanamycin, and dithiothreitol (DTT) were purchased from Fisher BioReagents, Daejung Chemicals, and BioBasic, respectively. Ni-NTA agarose was purchased from Qiagen. HEPES and LB broth were purchased from Dojindo Laboratories and Gibco, respectively.

For matrix-associated laser desorption ionization time-of-flight (MALDI-TOF) analysis, sinapic acid (SA), α-cyano-4-hydroxycinnamic acid (CHCA), and 2,5-dihydroxybenzoic acid (DHB) were purchased from Sigma-Aldrich.

For high-performance liquid chromatography (HPLC) analysis, HPLC-grade acetonitrile (ACN), formic acid, and trifluoroacetic acid (TFA) were purchased from Samchun Chemicals. 4-Nitrobenzenesulfonamide and 4-nitrobenzyl bromide were purchased from Combi-Blocks. 5(6)-Carboxyfluorescein was purchased from Sigma-Aldrich, and pyridine-4-carboxaldoxime, 4-bromo-1-butyne, and 6-chloro-1-hexyne were purchased from Alfa Aesar. 2-Azidoacetic acid and *N*-succinimidyl D-biotinate were purchased from TCI. Fmoc-Val-Cit-PABC-PNP, monomethyl auristatin E (MMAE), and BCN-PEG₄-acid were purchased from Angene, Chemscene, and BLD Pharm, respectively.

For antibody labeling experiments, trastuzumab was purchased from Roche, and IdeS enzyme was from purchased ACROBiosystems. Sequencing-grade modified trypsin was purchased from Promega.

For DNA manipulation, PrimeSTAR HS DNA Polymerase and restriction enzymes were purchased from Takara Bio. DNA oligonucleotides were purchased from Macrogen, and PCR/Gel purification kits and plasmid mini-preparation kits were purchased from Nucleogen. For protein preparation, Amicon^{®} Ultra centrifugal filters and ZipTip^{®} pipette tips were purchased from Merck Millipore. Pierce^{™} C18 spin columns and Slide-A-Lyzer^{™} dialysis cassettes were purchased from Thermo Fisher Scientific, and a PD SpinTrap G-25 was purchased from Cytiva.

### 1.2. Analytical Methods

### 1) Analytical-High-Performance Liquid Chromatography (HPLC)

For reversed-phase liquid chromatography (RPLC), each sample (3 µg) was analyzed using an Agilent 1260 Infinity I system with a Poroshell 300SB-C8 column (Agilent, 1.0 x 75 mm, 5 µm) at a flow rate of 1 mL/min. Initially, mobile phase A (0.1% TFA in water) was maintained at 80% for 2 minutes. Mobile phase B (0.1% TFA in ACN) was increased from 20% to 60% over 2 to 8 minutes. Over an additional 2 minutes (8 to 10 minutes), mobile phase B was increased from 60% to 98% to elute all samples from the column, and detection was performed using a variable wavelength detector (VWD) at 280 nm.

For hydrophobic interaction chromatography (HIC), each sample (10 µg) was analyzed using the same HPLC as the MAbPac HIC-Butyl HPLC (Thermo Scientific^{™}, 4.6 x 100 mm, 5 µm) at a flow rate of 1 mL/min. A gradient from 0% to 100% was applied from 1 to 15 minutes. Buffer A: (1.5 M ammonium sulfate, 50 mM sodium phosphate, pH 7.0; Buffer B: (50 mM sodium phosphate, pH 7.0); Flow rate = 1 mL/min. The sample was detected using a VWD at 280 nm.

### 2) Mass Analysis of Fc Fragments and Trastuzumab

Each sample (30 µg) was washed with a PD SpinTrap G-25 equilibrated with ammonium bicarbonate buffer (50 mM) and treated with 30 units of IdeS enzyme prior to analysis. Samples were analyzed using a linear ion-trap Orbitrap mass spectrometer coupled to an Ultimate 3000 Ultra-High-Performance Liquid Chromatography (UHPLC, Thermo Fisher Scientific) with an analytical ACQUITY UPLC Protein BEH C4 column (Waters, 300 Å, 2.1 x 50 mm, 1.7 µm). For the UHPLC method, mobile phase A (0.1% TFA in water) was maintained at 80% for 1 minute initially. Mobile phase B (0.1% TFA in ACN) was increased from 20% to 90% over 1 to 10 minutes. Detection was performed using an Orbitrap MS over an extended mass range of 500 m/z to 4,000 m/z. Peak deconvolution was performed using UniDec software.

### 3) ADC Purification using HIC

To purify unpurified trastuzumab-MMAE, a HIC column (0.8 x 10 cm, 5 mL) prepacked with Skillpak Phenyl-650S HIC resin (Tosoh Bioscience) was connected to an AKTA pure (Cytiva). The entire process was performed at room temperature. Prior to sample injection, the column was equilibrated with 20 column volumes of Buffer A (50 mM sodium phosphate, pH 7.0, 2 M NaCl). To load the column, 0.25 mL of unpurified ADC (1 mg/mL in formulation buffer) was mixed with 0.25 mL of Buffer A. The resulting mixture (total volume 0.5 mL) was then injected into the column and eluted using a linear gradient from 100% Buffer A to 100% Buffer B (50 mM sodium phosphate, pH 7.0, 20% IPA (v/v)).

### 4) Nuclear Magnetic Resonance (NMR)

¹H NMR spectra were recorded at ambient temperature using a Bruker 400 MHz spectrometer and processed using MestReNova 6.0.2 software with automatic phase correction and polynomial baseline correction, or recorded on a Varian 400 MHz spectrometer. Typical ¹³C NMR spectra were recorded on Bruker 400 MHz or Bruker 700 MHz spectrometers with complete proton decoupling. ¹³C chemical shifts were reported in ppm relative to the residual solvent peak of CDCl₃ (77.16 ppm).

### 5) Fourier-Transform Infrared Spectroscopy (FTIR)

Infrared spectra were recorded on a JASCO FT/IR-4600 spectrometer, with νₘₐₓ values partially reported in cm⁻¹. High-resolution mass spectra were obtained using a JEOL JMS-700 instrument equipped with FAB mode. Analytical thin-layer chromatography was performed using 60 Å silica gel F254 precoated plates (0.25 mm thick), and the TLC plates were visualized under UV lamp illumination. Normal-phase column chromatography was performed on an Agilent 1260 series instrument equipped with a COSMOSIL diode array detector and column, using 60 Å silica gel (32 to 62 µm) with an appropriate mobile phase composition and gradients

### 2. Experimental Procedures

### 2.1. Protein Production

### 1) Protein Expression, ncAA Incorporation, and Purification in Escherichia coli

To obtain strep-tagged Z-domain protein (STZ), the wild-type (WT) Z-domain gene was amplified through gene synthesis, and overlapped with the N-terminal strep-tag sequence (GWSHPQFEK) and C-terminal hexa-histidine tag sequence to generate the STZ gene. Using the NdeI and EcoRI restriction sites and the pET20b vector, pET20b-strep Z-domain was constructed. Then, pET20b-Strep Z-domain was transformed into *Escherichia coli* BL21(DE3) cells and cultured overnight at 37°C in 6 mL of LB broth containing ampicillin (100 µg/mL). The cultured cells (1 mL) were inoculated into a defined medium (50 mM Na₂HPO₄, 50 mM KH₂PO₄, 25 mM (NH₄)₂SO₄, 2 mM MgSO₄, 0.1% trace metals, 0.5% glycerol, 0.05% glucose, 0.2% lactose, and 5% amino acids) supplemented with the same concentration of ampicillin and cultured overnight at 37°C. Cells were harvested by centrifugation (10,000 rpm, 5 minutes), and the cell pellet was frozen at -20°C for further purification. According to the manufacturer's protocol, the pellet was thawed and purified using Ni-NTA agarose resin. The concentration of the purified protein was measured by UV absorbance at 280 nm, and purity was assessed by 8% to 12% SDS-PAGE. The molar extinction coefficient of the protein was calculated using the Biomol Protein Extinction Coefficient Calculator (http://www.biomol.net/en/tools/proteinextinction.htm).

The affibody was obtained through gene synthesis, and a hexa-histidine tag was attached using overlap PCR. The gene of the Z-M variant was generated from a minimized Z-domain of protein A, with an additional sequence corresponding to the third helix of the WT Z-domain. Additionally, a hexa-histidine tag was added to the C-terminal of the gene. The affibody gene was inserted into the pBAD vector at the NdeI/EcoRI restriction sites to generate pBAD-affibody, and the Z-M variant gene was inserted into the pET20b vector at the NcoI/KpnI restriction sites to generate pET20b-Z-M. Site-directed mutagenesis was performed to produce pBAD-affibody variants (K4, K28, F32TAG) and pET20b-Z-M variants (M8, H19, K33, D37TAG). For pBAD-affibody F32TAG, pET20b-Z-M M8, and D37TAG, the closest Lys was substituted with Arg (CGT codon) to prevent self-labeling of the probe, generating K28CGT_F32TAG, K5CGT_M8TAG, and K33CGT_D37TAG, respectively. The plasmids were then co-transformed into DH10B or BL21 cells with pEvol-AzFRS or pEvol-AzKRS. Transformed cells were expressed and purified as described above using unnatural amino acids (AzF at 100 µM or AzK at 1 mM) and chloramphenicol (35 µg/mL). Concentration and purity were also tested in the same manner.

### 2) Expression and Purification of IgG1 Fc Domain

The Fc domain gene was amplified by PCR and inserted between the XhoI and BamHI sites of the pCDNA3.4 vector (Invitrogen, USA) to generate pCDNA3.4-Fc domain. The plasmid containing the Fc domain was transiently transfected into FreeStyle^{™} 293 cells (Invitrogen, USA) using PEI transfection reagent according to the manufacturer's protocol (Polysciences, USA). For expression, the supernatant from FreeStyle^{™} 293 cells transiently transfected with each Fc domain construct was harvested by centrifugation at 8,000 rpm for 20 minutes at 4°C. The expressed Fc domain was purified using a Hitrap Protein A HP column according to the manufacturer's protocol (Cytiva, Switzerland), and the column was equilibrated with Buffer A (1.8 mM KH₂PO₄, 10 mM K₂HPO₄, 137 mM NaCl, 2.7 mM KCl, pH 7.4). The supernatant containing the Fc domain was loaded onto the column, washed to remove unbound impurities, and the purified Fc domain was eluted with Buffer B (100 mM citrate, 100 mM NaCl, pH 3.0). The purified Fc domain was dialyzed three times against Buffer A to remove any residual elution buffer. The concentration of the Fc domain was determined by measuring absorbance at 280 nm using the molar extinction coefficient (7.157 × 10⁴ cm⁻¹ M⁻¹) of the Fc domain.

### 3) Copper(I)-Catalyzed Azide-Alkyne Cycloaddition (CuAAC)

Purified affibody and Z-M protein variants were dialyzed against 20 mM potassium phosphate buffer (100 mM NaCl, pH 7.0) using a Slide-A-Lyzer^{™} dialysis cassette, 3.5K MWCO. A mixture of 20 µM of each protein, PyOx (4 mM), CuSO₄:BTTAA premix (300 µM:1,500 µM), sodium-L-ascorbate (5 mM), and aminoguanidine hydrochloride (5 mM) was incubated at 25°C with stirring at 250 rpm for 1 hour. The reaction mixture was dialyzed against 50 mM HEPES buffer (pH 7.2 or 8.0) and concentrated using an Amicon^{®} Ultra centrifugal filter (3K MWCO) for further reactions.

### 2.2. Protein Labeling

### 1) Labeling of NASA-FL with STZ

Affibody variants containing PyOx (30 µM) were mixed with STZ (15 µM). NASA-FL (75 µM) was added to the mixture, and the resulting mixture was incubated in HEPES buffer (50 mM, pH 7.2) at 37°C for 4 hours. The reaction was quenched with a 100 mM mixture of L-Lys and But-PyOx, resulting in final concentrations of 5 mM each in the reaction mixture. The labeled NASA-FL was desalted using a ZipTip containing 0.6 µL C18 resin according to the manufacturer's protocol. Labeling yield was determined by MALDI-TOF MS analysis (matrix: sinapic acid).

### 2) Fc Fragment Labeling with NASA-FL for PyOx Site Selection in Z-M Protein Variants

Z-M variants containing PyOx (30 µM) were mixed with the Fc fragment (7.5 µM). NASA-FL (75 µM) was also added to the mixture, and the resulting mixture was incubated in HEPES buffer (50 mM, pH 7.2) at 37°C for 4 hours, followed by quenching in the same manner as described above. Unreacted NASA-FL was removed by washing with a PD SpinTrap G-25.

### 3) Optimized Labeling of NASA Probe for Fc Fragment and Trastuzumab

Z-M variants containing PyOx (60 µM) were mixed with Fc fragment or trastuzumab (5 µM). NASA-probe (250 µM) was added to the mixture, and the resulting mixture was incubated in HEPES buffer (50 mM, pH 8.0) at 37°C for 6 hours, followed by quenching in the same manner as described above. Unreacted NASA probe was removed by washing with a PD SpinTrap G-25.

### 4) ADC Preparation

To prepare ADC, trastuzumab (5 µM) was mixed with Z-M M8AzK-PyOx (60 µM). NASA-N₃ (60 µM) was added to the mixture, and the mixture was incubated in HEPES buffer (50 mM, pH 8.0) at 37°C for 6 hours to generate trastuzumab-N₃. After the reaction, the mixture was quenched and buffer-exchanged with PBS (pH 7.4) for further SPAAC reaction. Trastuzumab-N₃ (10 µM) was mixed with BCN-MMAE (50 µM) and reacted at 25°C for 36 hours. The product was buffer-exchanged with PBS for further purification using FPLC.

### 2.3. Analysis of Labeled Residues

### 1) Digestion of WT and FL-Labeled STZ

WT and FL-labeled STZ (15 µg) were washed with a PD SpinTrap G-25 equilibrated with ammonium bicarbonate buffer (50 mM). Urea (final concentration: 6 M) was added to the reaction mixture and the resulting mixture was incubated at 50°C for 45 minutes. Endoproteinase Glu-C (Worthington) was added at a final concentration of 2 µM, and the mixture was incubated overnight at 37°C. The reaction mixture was quenched with 0.4% TFA and further purified using a Pierce^{™} C18 spin column. The purified product was concentrated by solvent evaporation under N₂ blowing and mixed evenly with DHB. MALDI-TOF (Bruker Autoflex^{®}) was used to identify labeled peptide fragments, and MALDI-TOF/TOF was employed to determine the exact labeled residues.

### 2) Digestion of WT and Probe-Labeled Fc Fragment

WT and probe-labeled Fc fragment (30 µg) were washed with a PD SpinTrap G-25 equilibrated with ammonium bicarbonate buffer (50 mM) containing 0.1% SDS. DTT was added at a final concentration of 20 mM, and the mixture was incubated at 95°C for 20 minutes. Subsequently, IAA was added to a final concentration of 50 mM, the mixture was incubated in the dark at 25°C in for 30 minutes. Excess IAA was quenched with 5 mM DTT. Sequencing-grade modified trypsin was added at a ratio of 1/20 relative to the Fc fragment, and the mixture was incubated overnight at 37°C. The reaction mixture was quenched with 0.4% TFA and purified using a C18 spin column. The sample was further concentrated by solvent evaporation under N₂ blowing and mixed evenly with CHCA for analysis. MALDI-TOF was employed to detect labeled peptide fragments, and MALDI-TOF/TOF was employed to identify the exact labeled residues.

### 3) Digestion of WT and Probe-Labeled Antibodies

WT and probe-labeled trastuzumab (30 µg) were washed with a PD SpinTrap G-25 equilibrated with ammonium bicarbonate buffer (50 mM) and treated with 30 units of IdeS enzyme. Then, 0.1% SDS was added to the samples, and sequencing-grade modified trypsin was added at a ratio of 1/20 relative to the antibody. Sample preparation for MALDI-TOF and MALDI-TOF/TOF was performed in the same manner as for Fc fragment analysis.

### 2.4. Binding Affinity Measurement Using Surface Plasmon Resonance (SPR)

### 1) Binding Affinity Analysis of Z-M Protein and Antibody

The binding constant (K_{D}) was measured via a His capture method using an amine-coupled CM5 chip. HBS-EP+ buffer (150 mM NaCl, 10 mM HEPES, 3 mM EDTA, and 0.05% (v/v) surfactant P20, pH 7.4) was used as the running buffer. A blank channel of the chip served as a negative control, and the affibody was immobilized on the chip. Two-fold serial dilutions of the antibody at various concentrations (0.78 nM, 1.56 nM, 3.13 nM, 6.25 nM, and 12.50 nM) were flowed over the chip surface. After each cycle, the chip surface was regenerated with 10 mM glycine-HCl buffer (pH 1.5), and affinities were calculated with BIA evaluation software using a 1:1 (Langmuir) binding fit model.

### 2) Binding Affinity Analysis of FcRn (Neonatal Fc Receptor) with Antibody or ADC

To evaluate binding kinetics, surface plasmon resonance (SPR) analysis was performed using a BIAcore T200 system. The binding constant (K_{D}) was determined via a direct immobilization method using an amine-coupled CM5 chip. Phosphate buffer (50 mM sodium phosphate, 150 mM NaCl, and 0.05% (v/v) surfactant P20) at pH 6.0 or pH 7.4 was used as the running buffer. The blank channel of the chip served as a negative control, and FcRn was immobilized on the chip. Two-fold serial dilutions of the antibody at various concentrations (1.96 nM, 3.91 nM, 7.81 nM, 15.63 nM, and 31.25 nM) were flowed over the chip surface. After each cycle, the chip surface was regenerated with 0.1 M Tris-HCl buffer (pH 8.0), and affinity were calculated with BIA evaluation software using a 1:1 (Langmuir) binding fit model.

### 3) Binding Affinity Analysis of Human Epidermal Growth Factor Receptor Type 2 (HER2) with Antibody or ADC

The binding constant (K_{D}) was determined via the human Fc method using an amine-coupled CM5 chip. HBS-EP+ buffer (150 mM NaCl, 10 mM HEPES, 3 mM EDTA, and 0.05% (v/v) surfactant P20, pH 7.4) was used as the running buffer. A blank channel of the chip served as a negative control, and trastuzumab or trastuzumab-ADC was immobilized on the chip. Two-fold serial dilutions of the antibody at various concentrations (0.78 nM, 1.56 nM, 3.13 nM, 6.25 nM, and 12.50 nM) were flowed over the chip surface. After each cycle, the chip surface was regenerated with 3 M magnesium chloride buffer, and affinities were calculated with BIA evaluation software using a 1:1 (Langmuir) binding fit model.

### 2.5. Cytotoxicity Evaluation of ADC

### 1) Cell Culture

MDA-MB-231, MDA-MB-453, and SK-BR-3 were purchased from KCLB (Seoul, Korea). Cells were cultured in RPMI 1640 supplemented with 10% (v/v) fetal bovine serum (FBS) and 1% (v/v) penicillin/streptomycin (Welgene, Korea) and incubated at 37°C in a humidified 5% CO₂ incubator.

### 2) Anti-proliferation Assay

Cells were seeded in 96-well plates at a density of 3,000 cells per well. After cell attachment, cells were treated with ADC serially diluted four-fold in PBS. After incubation at 37°C for 72 hours, cell viability was measured using CellTiter-Glo (G7572, Promega, Madison, WI, USA). Fitter dose-response curves and GI₅₀ values were obtained using GraphPad Prism 6.0 software, and all experiments were conducted in duplicate.

### 3) Western Blot

Primary antibodies for HER2 (#2165) and GAPDH (#5174) were purchased from Cell Signaling Technology. HRP-conjugated goat anti-rabbit secondary antibody (SA002-500) was purchased from Gendepot. Each cell was washed twice briefly with cold PBS and lysed using NP40 buffer (50 mM Tris-HCl pH 7.4, 1% NP40, 2 mM EDTA, 150 mM NaCl) supplemented with a protease inhibitor cocktail (#11873580001, Roche) and a phosphatase inhibitor cocktail (#04906837001, Roche). Equal amounts of protein from each sample were loaded and separated on an SDS-PAGE gel. The samples were transferred onto a nitrocellulose membrane and blocked with 5% skim milk (TBS/T). The membrane was incubated overnight at 4°C with the primary antibody, uniformly diluted 1:1,000 (v/v) in TBS/T. After incubation with secondary antibody (1:10,000 v/v) for 1 hour at room temperature, the membrane was treated with ECL solution, and chemiluminescent signals were detected using an ImageQuant^{™} LAS 4000 (GE Healthcare, USA). The western blot images were quantified using ImageJ (n = 3).

### 3. Synthesis Methods of Each Compound

### 3.1. Synthesis of PyOx-Alkyne

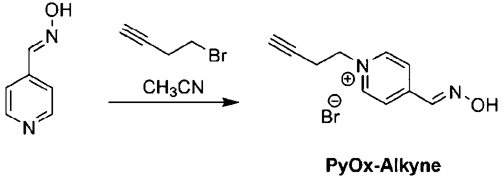

To a stirred solution of pyridine-4-carboxaldoxime (370 mg, 3.03 mmol, 1 equiv) in dry CH₃CN (1.5 mL), 4-bromo-1-butyne (0.60 mL, 6.6 mmol, 2.2 equiv) was added, and the mixture was refluxed for 24 hours. The solution was cooled to room temperature and filtered to afford PyOx-alkyne (518 mg, 2.03 mmol, 67%) as a yellow solid.

¹H NMR (400 MHz, D₂O) δ 8.86 (d, *J* = 6.4 Hz, 2H), 8.24 (s, 1H), 8.15 (d, *J =* 6.4 Hz, 2H), 4.64 (t, *J =* 6.4 Hz, 2H), 2.88 (m, 2H), 2.48 (m, 1H).

HRMS: Exact mass calculated for [C₁₀H₁₁N₂O₁ + H]⁺ requires *m*/*z* = 175.0866, found *m*/*z* = 175.0868 (HESI⁺).

### 3.2. Synthesis of PyOx_C₆-Alkyne

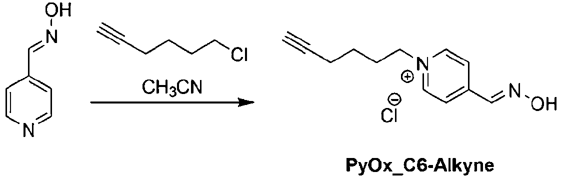

To a stirred solution of pyridine-4-carboxaldoxime (370 mg, 3.03 mmol, 1 equiv) in dry CH₃CN (1.5 mL), 6-chloro-1-hexyne (0.73 mL, 6.06 mmol, 2.2 equiv) was added. The mixture was heated to reflux (85°C, vial) and stirred for 25 hours. The precipitate was collected and stirred in dry CH₃CN (1.5 mL) for an additional 24 hours. The solution was cooled to room temperature and filtered to afford PyOx_C₆-alkyne (210 mg, 1.04 mmol, 34%) as a yellow solid.

¹H NMR (400 MHz, D₂O) δ 8.80 (d, *J =* 6.4 Hz, 2H), 8.34 (s, 1H), 8.16 (d, *J* = 6.4 Hz, 2H), 4.59 (t, J = 7.6 Hz, 2H), 2.32 (m, 1H), 2.25 (m, 2H), 2.11 (m, 2H), 1.55 (m, 2H).

HRMS: Exact mass calculated for [C₁₂H₁₅N₂O₁ + H]⁺ requires *m*/*z* = 203.1179, found *m*/*z =* 203.1182 (HESI⁺).

### 3.3. Synthesis of tert-butyl (15-((4-nitrophenyl)sulfonamido)-15-oxo-3,6,9,12-tetraoxapentadecyl) carbamate (S1)

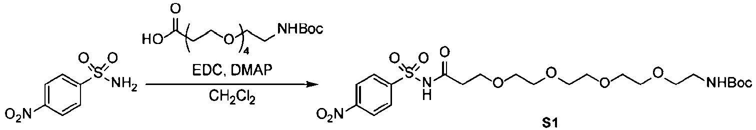

To a solution of Boc-NH-PEG₄-COOH (1.00 g, 2.74 mmol, 1.2 equiv) in dry DMF (10 mL), diisopropylethylamine (1.05 mL, 6.02 mmol), 4-nitrobenzenesulfonamide (0.61 g, 3.01 mmol, 1 equiv), and 1-[bis(dimethylamino)methylene]-1H-1,2,3-triazolo[4,5-b]pyridinium 3-oxide hexafluorophosphate (HATU) (1.25 g, 3.28 mmol) were added, and the mixture was stirred at room temperature for 6 hours. The resulting mixture was diluted with ethyl acetate and washed twice with 0.5 M aqueous HCl solution and brine. The organic phase was dried over MgSO₄ and evaporated under reduced pressure. The residue was purified by flash column chromatography on silica gel (CH₂Cl₂:MeOH = 20:1) to afford S1 (0.83 g, 55% yield) as a colorless oil.

¹H NMR (400 MHz, CDCl₃) δ 8.32 (d, *J* = 8.8 Hz, 2H), 8.25 (d, *J* = 8.8 Hz, 2H), 3.71-3.51 (m, 18H), 3.32 (m, 2H), 2.56 (m, 2H), 1.42 (s, 9H).

¹³C NMR (100 MHz, CDCl₃) δ 156.4, 150.3, 129.7 (2C), 123.9 (2C), 70.69, 70.62, 70.58, 70.5, 70.35, 70.32, 70.2, 70.11, 70.06, 66.7, 53.6, 40.4, 37.8, 28.5 (3C).

### 3.4. Synthesis of tert-butyl (1-(4-nitrophenyl)-2-((4-nitrophenyl)sulfonyl)-3-oxo-6,9,12,15-tetraoxa-2-azaheptadecan-17-yl)carbamate (S2)

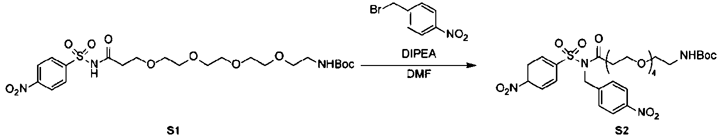

To a solution of compound S1 (1.0 g, 1.8 mmol, 1 equiv) in dry DMF (6.3 mL, 0.3 M), 4-nitrobenzyl bromide (472 mg, 2.2 mmol, 1.2 equiv) and DIPEA (0.6 mL, 3.6 mmol, 2 equiv) were added, and the mixture was stirred at room temperature for 24 hours. After removing the solvent, the reaction mixture was diluted with EtOAc and washed with brine. The organic layer was dried over MgSO₄ and evaporated under reduced pressure. The residue was purified by flash column chromatography on silica gel (hexane:EtOAc = 1:2) to afford compound S2 (731 mg, 59% yield) as a colorless oil.

¹H NMR (400 MHz, CDCl₃) δ 8.39 (d, *J* = 9.0 Hz, 2H), 8.24 (d, *J* = 8.8 Hz, 2H), 8.13 (d, *J* = 8.9 Hz, 2H), 7.56 (d, *J* = 8.8 Hz, 2H), 5.20 (s, 2H), 3.70-3.65 (m, 2H), 3.64-3.57 (m, 8H), 3.56-3.52 (m, 2H), 3.52-3.48 (m, 4H), 3.34-3.23 (m, 2H), 2.82 (t, *J* = 6.0 Hz, 2H), 1.43 (s, 9H) .

¹³C NMR (175 MHz, CDCl₃) δ 171.5, 151, 147.9, 144.7, 143.4, 129.5 (2C), 128.4 (2C), 124.6 (2C), 124.2 (2C), 70.8, 70.7 (2C), 70.5, 70.39, 70.37, 66.5, 49.5, 40.5, 37.0, 31.7, 28.6 (3C), 22.8, 14.2.

### 3.5. Synthesis of NASA-FL

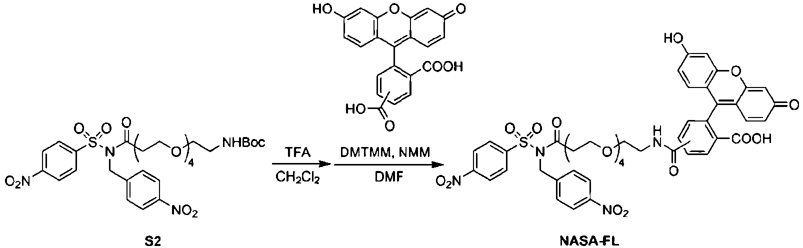

To a solution of compound S2 (8.2 mg, 12.0 µmol, 1 equiv) in CH₂Cl₂ (0.5 mL), TFA (30 µL) was added and stirred at room temperature for 1 hour. The solvent was removed under vacuum. To a solution of this residue in dry DMF (0.5 mL), 5(6)-carboxyfluorescein (5.4 mg, 14.4 µmol, 1.2 equiv), 4-(4,6-dimethoxy-1,3,5-triazin-2-yl)-4-methylmorpholinium chloride (DMTMM) (3.9 mg, 14.1 µmol, 1.2 equiv), and N-methylmorpholine (NMM) (3.9 µL, 35.5 µmol, 3 equiv) were added. The mixture was stirred at room temperature for 1 hour and then overnight at 4°C. After removing the solvent, the resulting mixture was purified by flash column chromatography on silica gel (CH₂Cl₂:MeOH = 10:1) to afford compound NASA-FL (8.5 mg, 75% yield).

¹H NMR (400 MHz, CD₃OD) δ 8.39 (d, *J* = 8.4 Hz, 2H), 8.20-8.10 (m, 6H), 7.61-7.22 (m, 3H), 6.66-6.50 (m, 6H), 5.25 (s, 2H), 3.63-3.33 (m, 18H), 2.79-2.75 (m, 2H).

HRMS: Exact mass calculated for [C₄₅H₄₂N₄O₁₇S + H] ⁺ requires *m*/*z* = 943.2338, found *m*/*z* = 943.2339 (HESI⁺).

### 3.6. Synthesis of NASA-Bt

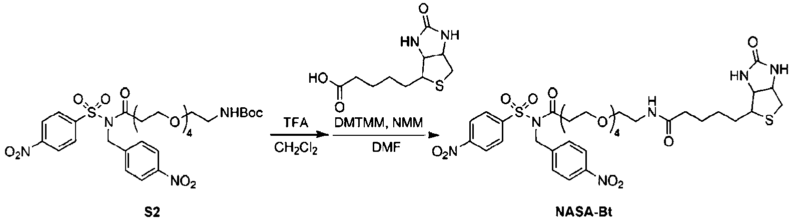

To a solution of compound S2 (8.2 mg, 12.0 µmol, 1 equiv) in CH₂Cl₂ (0.5 mL), TFA (30 µL) was added. The mixture was stirred at room temperature for 1 hour, and the solvent was removed under vacuum. The resulting residue was dissolved in dry DMF (0.5 mL), and N-succinimidyl D-biotinate (4.92 mg, 14.4 µmol, 1.2 equiv), DMTMM (3.9 mg, 14.1 µmol, 1.2 equiv), and N-methylmorpholine (NMM) (3.9 µL, 35.5 µmol, 3 equiv) were added thereto. The resulting mixture was stirred at room temperature for 1 hour and then overnight at 4°C. After removing the solvent, the resulting mixture was purified by flash column chromatography on silica gel (CH₂Cl₂:MeOH = 10:1) to afford compound NASA-Bt (5.4 mg, 55% yield).

¹H NMR (400 MHz, CD₃OD) δ 8.34 (d, *J* = 8.4 Hz, 2H), 8.15 (m, 4H), 8.11 (d, *J* = 8.9 Hz, 2H), 7.53 (d, *J* = 8.8 Hz, 2H), 5.20 (s, 2H), 4.38 (m, 1H), 4.19 (m, 1H), 3.56-3.38 (m, 18H), 3.09 (m, 1H), 2.76 (m, 2H), 2.59 (dd, *J* = 12.8 Hz, 1H), 2.10 (m, 2H), 1.55-1.32 (m, 6H).

HRMS: Exact mass calculated for [C₃₄H₄₆N₆O₁₃S₂ + Na]⁺ requires *m*/*z* = 833.2457, found *m*/*z =* 833.2490 (HESI⁺).

### 3.7. Synthesis of NASA-N₃

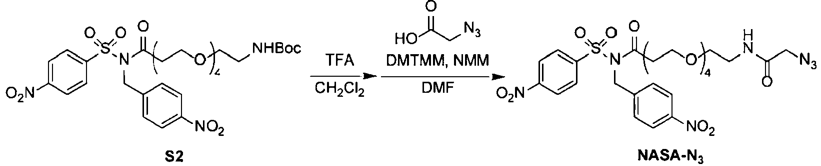

To a solution of compound S2 (100 mg, 0.15 mmol, 1 equiv) in CH₂Cl₂ (7 mL, 0.02 M), TFA (0.02 mL, 0.30 mmol, 2 equiv) was added. The mixture was stirred at room temperature for 1 hour, and the solvent was removed under vacuum. The resulting residue was dissolved in dry DMF (7 mL, 0.02 M), and 2-azidoacetic acid (18 mg, 0.18 mmol, 1.2 equiv), DMTMM (49 mg, 0.18 mmol, 1.2 equiv), and *N-*methylmorpholine (NMM) (0.05 mL, 0.35 mmol, 2.3 equiv) were added thereto. The resulting mixture was stirred at room temperature for 8 hours. After removing the solvent, the resulting mixture was purified by flash column chromatography on silica gel (CH₂Cl₂:MeOH = 20:1) to afford compound NASA-N₃ (83 mg, 86% yield) as a colorless oil.

¹H NMR (400 MHz, CDCl₃) δ 8.37 (d, J = 9.0 Hz, 2H), 8.20 (d, *J* = 8.8 Hz, 2H), 8.11 (d, *J* = 8.9 Hz, 2H), 7.54 (d, *J* = 8.7 Hz, 2H), 6.94 (s, 1H), 5.18 (s, 2H), 3.91 (s, 2H), 3.66 (t, *J =* 6.1 Hz, 2H), 3.63-3.57 (m, 8H), 3.56-3.46 (m, 6H), 3.43 (m, 2H), 2.81 (t, *J* = 6.1 Hz, 2H).

¹³C NMR (175 MHz, CDCl₃) δ 171.5, 166.9, 150.9, 147.9, 144.7, 143.4, 129.5 (2C), 128.4 (2C), 124.6 (2C), 124.2 (2C), 70.70, 70.66 (3C), 70.5, 70.4, 69.7, 66.5, 52.8, 49.5, 39.3, 37.0.

IR (FT-ATR, cm⁻¹, CH₂Cl₂) νₘₐₓ 3064, 2873, 2359, 2338, 2106, 1837, 1818, 1779, 1756, 1707, 1689, 1669, 1647, 1641, 1629, 1623, 1606, 1576, 1570, 1559, 1526, 1496, 1481, 1465, 1442, 1424, 1404, 1346, 1267, 1168, 1086, 1012, 854, 731, 700, 682.

HRMS: Exact mass calculated for [C₂₆H₃₃N₇O₁₂S + H]⁺ requires *m*/*z* = 668.1981, found *m*/*z =* 668.1980 (FAB⁺).

### 3.8. Synthesis of BCN-VC-PABC-MMAE

### 1) Synthesis of Fmoc-VC-PABC-MMAE

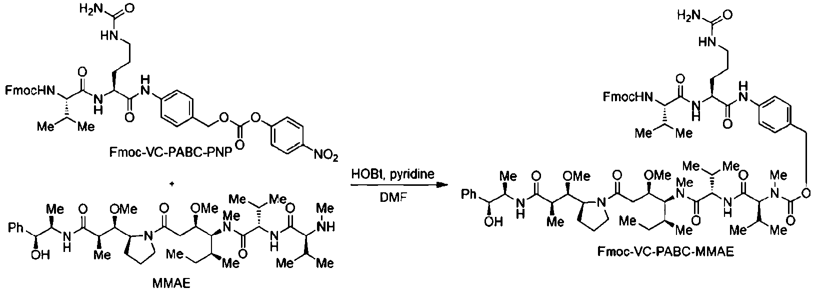

Fmoc-VC-PABC-PNP (200 mg, 0.26 mmol, 1 equiv) and MMAE (280 mg, 0.39 mmol, 1.5 equiv) were dissolved in DMF (40 mL, 6.5 mM). Then, hydroxybenzotriazole (HOBt) (8 mg, 0.52 mmol, 2 equiv) and pyridine (0.6 mL, 7.5 mmol, 28 equiv) were added to the solution. The mixture was stirred at room temperature for 30 hours and monitored by RP-HPLC. The product was purified by preparative column to afford a white powder (232 mg, 66% yield).

### 2) Synthesis of NH₂-VC-PABC-MMAE

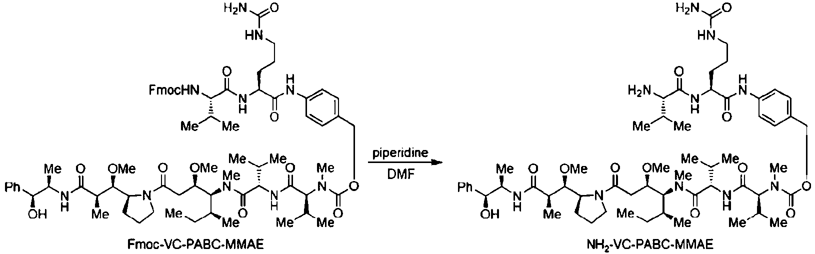

Fmoc-VC-PABC-MMAE (125 mg, 0.09 mmol) was dissolved in DMF (0.9 mL, 0.1 M) containing 20.0% piperidine and stirred at room temperature for 1 hour. After completion of the de-Fmoc process as confirmed by RP-HPLC analysis, the solution was concentrated under reduced pressure to afford a white powder (88.0 mg, 88% yield).

### 3) Synthesis of BCN-VC-PABC-MMAE

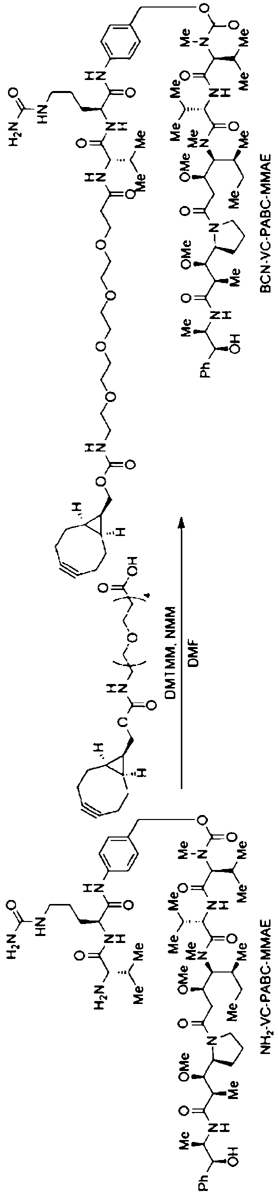

BCN-PEG₄-COOH (13 mg, 0.03 mmol, 1 equiv), NH₂-VC-PABC-MMAE (40 mg, 0.036 mmol, 1.2 equiv), DMTMM (10 mg, 0.036 mmol, 1.2 equiv), and *N*-methylmorpholine (NMM) (0.01 mL, 0.09 mmol, 3 equiv) were dissolved in DMF (0.14 mL, 0.2 M) and stirred at room temperature for 8 hours. After removing the solvent, the resulting mixture was purified by preparative column to afford a white powder (22 mg, 48% yield).

HRMS: Exact mass calculated for [C₈₀H₁₂₇N₁₁O₁₉ + Na]⁺ requires *m*/*z =* 1568.9202, found *m*/*z =* 1568.9223 (HESI⁺).

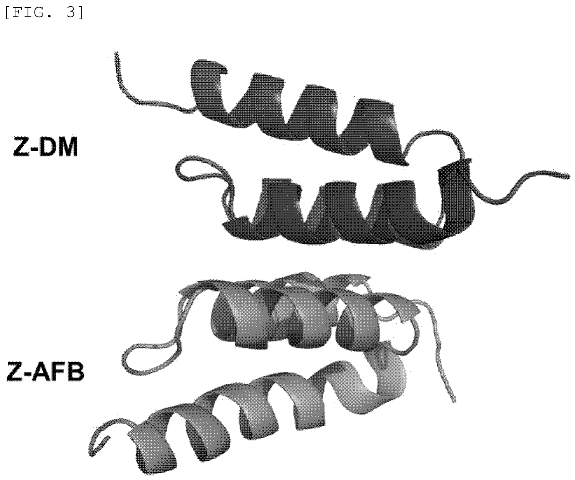

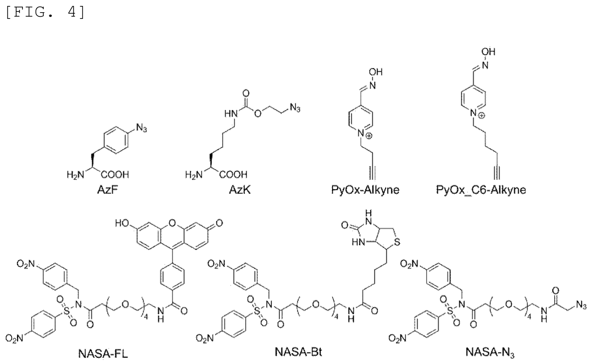

### 4. Site-Selective Labeling of Proteins (see FIGS. 3 and 4)

For site-selective labeling of intact proteins, a proximity-based labeling system employing a protein capable of binding to the binding protein was used. The binding protein acts as a catalyst, and the labeling reagent selectively reacts with the binding protein to form a highly reactive intermediate near the binding protein, thereby facilitating the formation of a covalent bond with a lysine residue adjacent to the binding protein.

As the target protein, a Z-domain protein (Z-DM), which is a variant of the B-domain of Staphylococcal Protein A, was used, and as the binding protein, a Z-domain-specific affibody (Z-AFB) was used *(see* FIG. 3). Both proteins are small (8 kDa or less) and have complex structures with high resolution, making them suitable as model systems. To ensure the catalytic function of the binding protein, pyridinium aldoxime (PyOx) was used as a catalyst capable of forming a highly reactive intermediate with the labeling reagent *(see* FIG. 4). PyOx is known to react rapidly with *N-*alkylsulfonamide (NASA) to form a reactive ester, which subsequently forms an amide bond with an amine with high efficiency.

To incorporate PyOx at specific sites of Z-AFB, the expansion technology known as genetic code expansion, which enables site-specific incorporation of an azide-containing ncAA into Z-AFB, was employed. Subsequently, Cu-catalyzed azide-alkyne cycloaddition (CuAAC) was used to attach PyOx to specific sites on Z-AFB. By simply mixing three components, that is, PyOx-containing Z-DM, Z-AFB, and NASA-derivatized probes, site-selective conjugation of probes to the binding protein was effectively achieved *(see* FIG. 5).

### 4.1. Labeling Experiments on Intact Z-DM

### 1) Incorporation of ncAA into Z-AFB

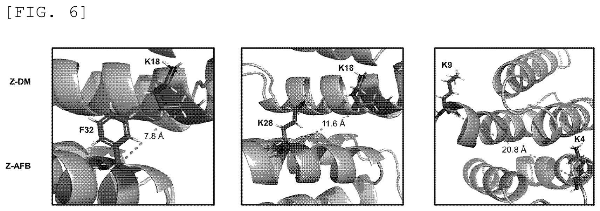

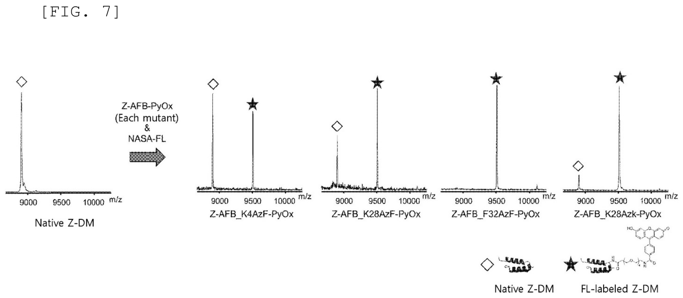

To incorporate PyOx at specific sites of Z-AFB, non-canonical amino acids (ncAA) were incorporated into Z-AFB. The ncAA incorporation sites (F32, K28, and K4) were selected based on the X-ray crystal structure of the Z-AFB and Z-DM complexes, and were positioned in proximity to Lys residues on Z-DM without affecting the binding of Z-AFB and Z-DM *(see* FIG. 6). 4-Azido-L-phenylalanine (AzF, *see* FIG. 7), which is widely used for protein labeling via bioorthogonal reactions, was used as the incorporated material. To incorporate AzF into Z-AFB, the codons corresponding to F32, K28, and K4 in the Z-AFB gene were modified to amber codons (TAG). The modified genes were expressed in the presence of AzF, along with the aminoacyl-tRNA and aminoacyl-tRNA synthetase (AzFRS) pair, resulting in the synthesis of Z-AFB variants containing AzF (Z-AFB_K4AzF, Z-AFB_K28AzF, and Z-AFB_F32AzF). Quantitative incorporation of AzF was confirmed through SDS-PAGE and MALDI-TOF MS analysis.

### 2) Incorporation of PyOx into AzF-Containing Z-AFB

PyOx was incorporated into AzF-containing Z-AFB through CuAAC reaction. Alkyne-substituted PyOx compounds *(see* Example 3.1) reacted with Z-AFB under previously reported conditions. SDS-PAGE and MALDI-TOF analysis confirmed the incorporation of PyOx into all Z-AFB variants (Z-AFB_K4AzF-PyOx, Z-AFB_K28AzF-PyOx, and Z-AFB_F32AzF-PyOx) .

### 3) Preparation of NASA-FL as Labeling Reagent

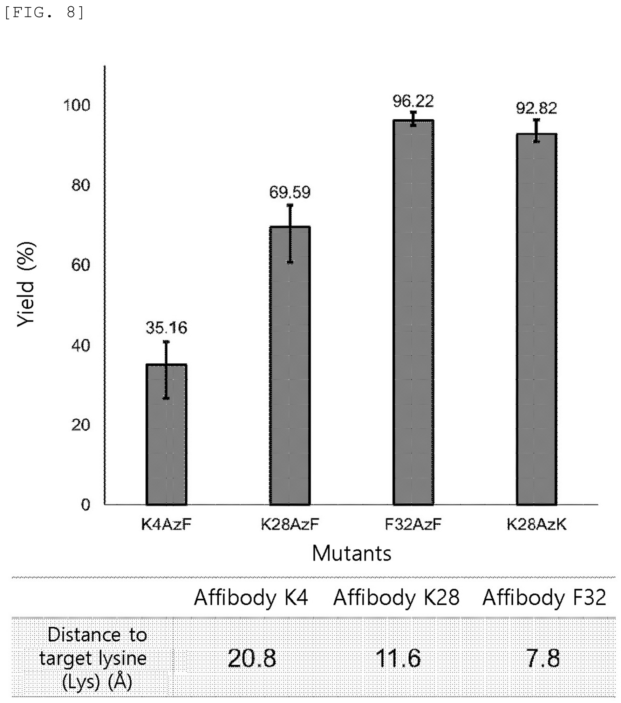

NASA-FL was synthesized as the labeling reagent by linking fluorescein (FL) and NASA using a previously reported method (*see* Example 3.5). By using Z-AFB-PyOx and NASA-FL, the feasibility of site-specific labeling of Z-DM in its native state was tested. Labeling experiments were conducted by simply mixing Z-DM, Z-AFB-PyOx, and NASA-FL. After the labeling reaction, the labeling efficiency was confirmed through MALDI-TOF analysis. The labeling reactions performed using the three Z-AFB-PyOx variants (Z-AFB_K4AzF-PyOx, Z-AFB_K28AzF-PyOx, and Z-AFB_F32AzF-PyOx) resulted in labeling efficiencies of 35%, 70%, and 96% for Z-AFB_K4AzF-PyOx, Z-AFB_K28AzF-PyOx, and Z-AFB_F32AzF-PyOx, respectively *(see* FIGS. 7 and 8).

The difference in labeling efficiency observed among the three variants correlated with the distance from the PyOx-incorporated site in Z-AFB to the nearest Lys residues on Z-DM; the shorter the distance, the higher the labeling efficiency. To verify this, Z-AFB_K28AzK-PyOx, which contains *N₆*-[(2-azidoethoxy)carbonyl]-L-lysine (AzK) with a longer alkyl chain instead of AzF, was synthesized and subjected to labeling experiments under same conditions, resulting in an enhanced labeling efficiency of 93% *(see* FIG. 7).

Collectively, the labeling method demonstrated high efficiency for labeling the native Z-DM protein (96% for labeling experiments using Z-AFB_F32AzF-PyOx). The labeling efficiency may be optimized by screening the site of PyOx using GCE technology and adjusting the length of the linker in Z-AFB-PyOx.

### 4.2. Identification of Labeled Residues in Z-DM.

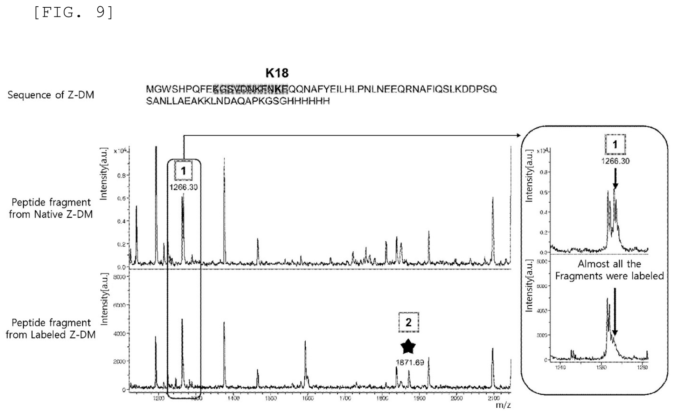

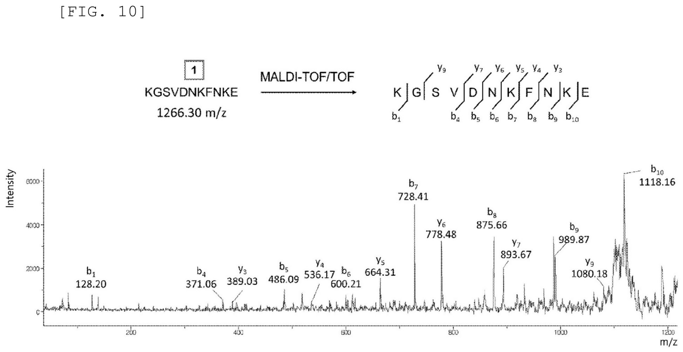

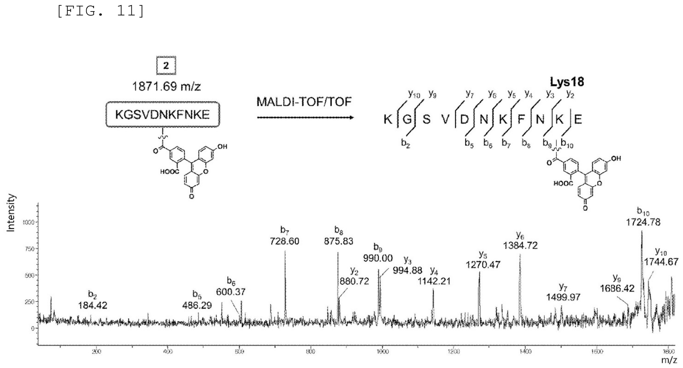

The labeled sites in Z-DM were confirmed to correspond with Lys residues predicted based on the crystal structure. In a labeling experiment using Z-AFB_F32AzF-PyOx, the FL-labeled Z-DM was treated with protease (Glu-C). Analysis of the generated peptides by MALDI-TOF/TOF tandem MS to determine the labeled sites confirmed that labeling had occurred at K18 of Z-DM, which corresponds to the position predicted based on the crystal structure (FIGS. 9 to 11). These results indicate that the designed labeling system can effectively label intact proteins and that the labeling site can be predicted through structural analysis.

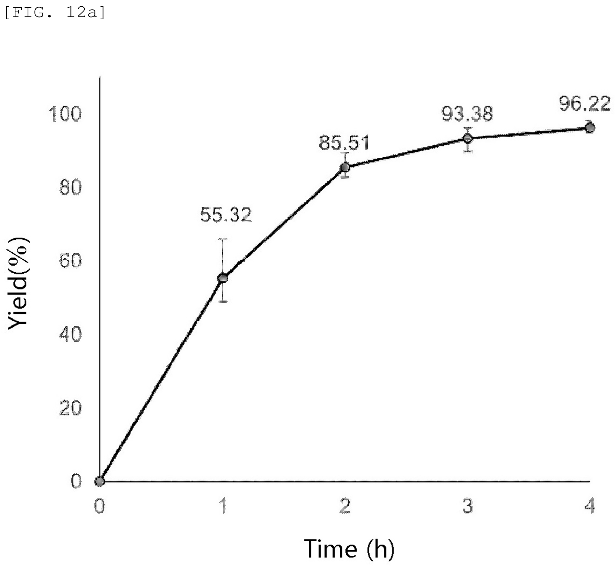

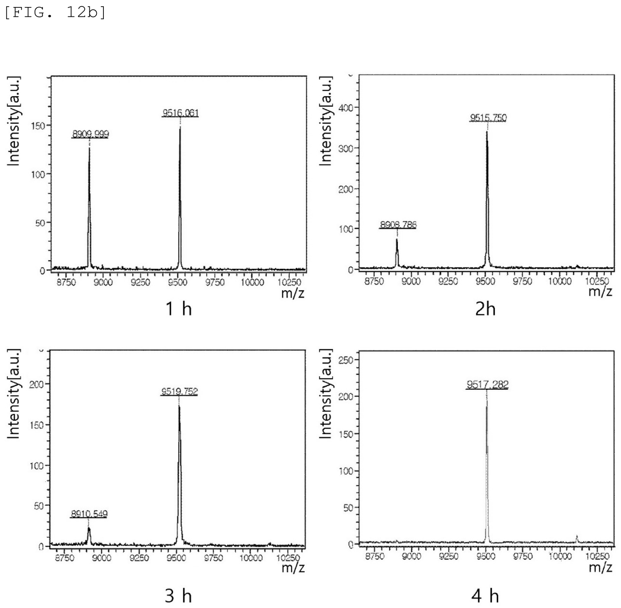

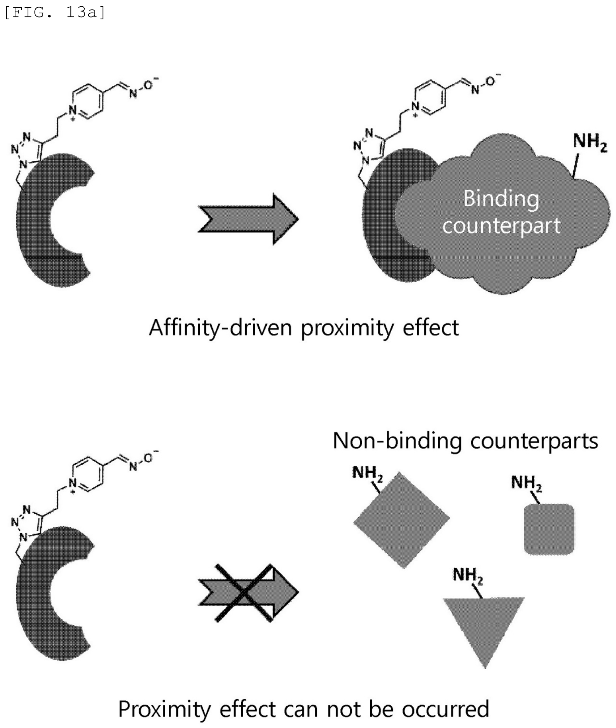

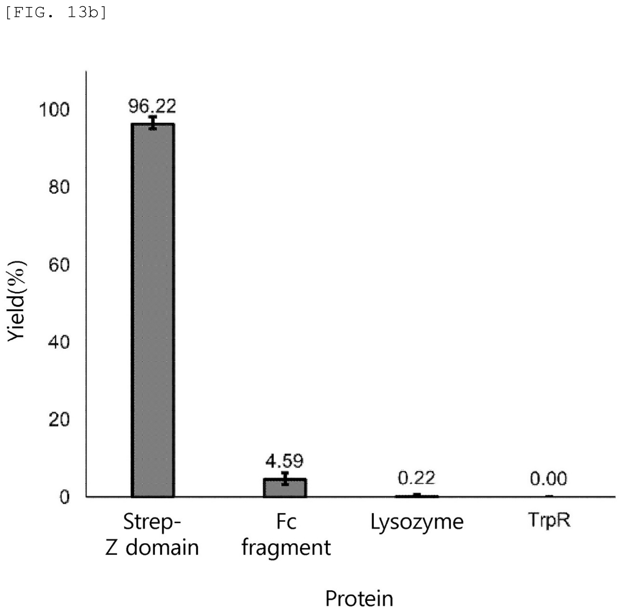

Furthermore, measurement of the labeling efficiency of Z-DM over various time points revealed that the efficiency increased over time for up to 4 hours after initiation of the reaction. More than half of the Z-DM was labeled within 1 hour, and 96% of the protein was labeled within 4 hours *(see* FIGS. 12a and 12b). When the reaction time was further extended, a small amount of labeling was observed at the N-terminal amine (data not shown); however, up to 4 hours, labeling was predominantly concentrated at K18. When the labeling reaction was applied to the unbound protein, no or negligible labeling occurred, indicating that the labeling reaction occurred only in the presence of the PyOx-containing binding protein *(see* FIGS. 13a and 13b).

### 4.3. System Design for Fc Fragment Labeling

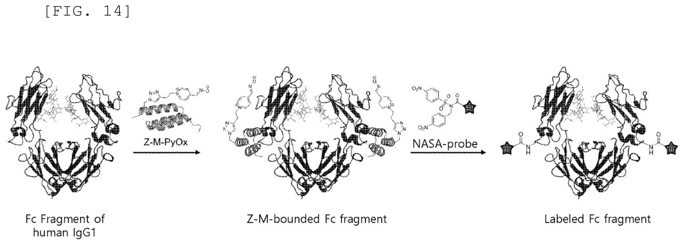

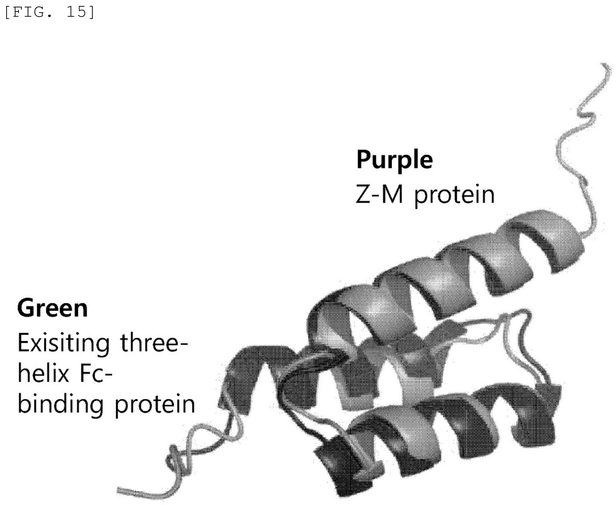

To apply the labeling system to antibody labeling, the Fc fragment of human immunoglobulin G1 (IgG1) was labeled *(see* FIG. 14). Because the Fc fragment is conserved across various therapeutic antibodies, the labeling method developed for the Fc fragment can be directly applied to antibodies containing the same Fc fragment. To achieve site-specific labeling of the Fc fragment, Fc-binding proteins with high affinity and selectivity were screened and evaluated, and a modified two-helix Fc-binding protein was selected, which was further optimized by introducing an additional helix for improved protein stability and expression. The additional helix design was based on the sequence of a previously reported three-helix Fc-binding protein. Computational analysis using the Alphafold2 program confirmed that the additional helix maintained the preferred three-helix structure (*see* FIG. 15), and the resulting three-helix protein (Z-M) was designed to stably and specifically bind the Fc fragment.

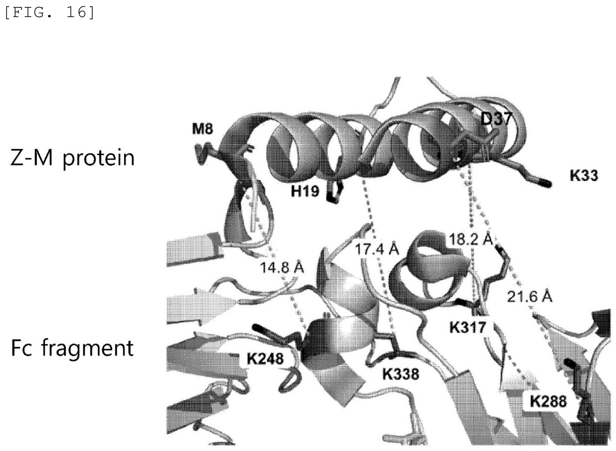

To determine suitable site for PyOx incorporation using the Z-M protein, which is an Fc-binding platform, the crystal structure of the complex formed by the two-helix Fc-binding protein and the Fc fragment was referenced. M8, H19, K33, and D37 of Z-M protein were selected as optimal sites for PyOx incorporation. These sites were determined as highly proximal to the Fc fragment with minimal disruption to Fc binding, enabling efficient labeling *(see* FIG. 16). The distances between the selected residue on Z-M and the nearest Lys on the Fc fragment were determined to be about 15 Å to about 22 Å, which was considerably longer than those measured for Z-DM/Z-AFB. Considering the correlation between labeling efficiency and the distance from the PyOx incorporation site to the nearest Lys, AzK, with its longer linker length compared to AzF, is expected to be more suitable for Fc fragment labeling.

Consequently, AzK was incorporated at sites M8, H19, K33, and D37 in Z-M using GCE technology, which was confirmed through SDS-PAGE and MALDI-TOF MS, indicating quantitative incorporation into the Z-M protein. Subsequently, PyOx was incorporated into each Z-M variant through CuAAC reaction, and quantitative conjugation was verified using SDS-PAGE and MALDI-TOF MS.

### 4.4. Binding Affinity Evaluation of Z-M Variants

The binding affinity of Z-M variants containing AzK or AzK-PyOx for antibodies with Fc domains (trastuzumab) were evaluated using surface plasmon resonance (SPR) analysis.

**[Table 1]**

| Samples | K_{D} (M) | Kₐ (1/Ms) | K_{d} (1/s) | Rₘₐₓ (RU) | Chi² (RU²) | U-value |
|---|---|---|---|---|---|---|
| Z-M WT | 4.964x10⁻⁹ | 1.897x10⁵ | 9.415x10⁻⁴ | 66.66 | 0.832 | 2 |
| Z-M_M8AzK | 3.939x10⁻⁹ | 1.820x10⁵ | 7.169x10⁻⁴ | 116.2 | 1.76 | 2 |
| Z-M H19AzK | 5.101x10⁻⁹ | 1.882x10⁵ | 9.597x10⁻⁴ | 62.52497 | 0.994 | 2 |
| Z-M K33AzK | 4.846x10⁻⁹ | 1.973x10⁵ | 9.561x10⁻⁴ | 70.48 | 0.948 | 2 |
| Z-M D37AzK | 3.521x10⁻⁹ | 1.941x10⁵ | 6.835x10⁻⁴ | 117.1 | 1.84 | 2 |
| Z-M_M8AzK-PyOx | 5.016x10⁻⁹ 9 | 1.914x10⁵ | 9.602x10⁻⁴ | 80.2403 | 0.963 | 2 |
| Z-M_H19AzK-PyOx | 4.944x10⁻⁹ | 1.934x10⁵ | 9.560x10⁻⁴ | 72.21 | 0.841 | 1 |
| Z-M_K33AzK-PyOx | 3.481x10⁻⁹ | 1.966x10⁵ | 6.843x10⁻⁴ | 118.3 | 2 | 2 |
| Z-M_D37AzK-PyOx | 4.792x10⁻⁹ | 2.002x10⁵ | 9.593 | 70.51 | 0.961 | 2 |

Referring to Table 1, all variants exhibited binding affinities comparable to that of the wild-type Z-M protein. This indicates that the incorporation of PyOx did not significantly affect the binding affinity between the Z-M protein variants and the Fc fragment.

### 4.5. Labeling of Intact Fc Fragment and Characterization of Labeled Residues

Labeling efficiency of the Fc fragment was tested using PyOx-containing Z-M variants. First, the optimal Z-M variant exhibiting the highest labeling efficiency was selected. The labeling reaction was performed by mixing each Z-M variant and NASA-FL with the Fc fragment, and labeling efficiency was assessed using HPLC analysis.

### 1) Analysis Results

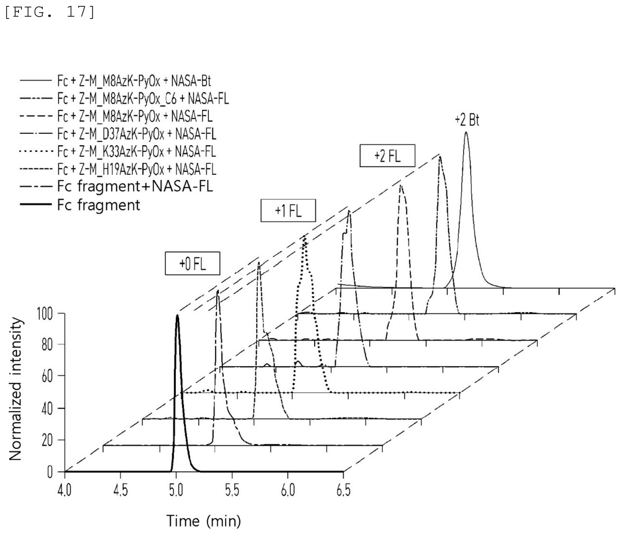

Z-M_M8AzK-PyOx exhibited the highest labeling efficiency and was shown to effectively and quantitatively label the Fc fragment, with negligible or no remaining unlabeled or partially labeled Fc *(see* FIG. 17). In contrast, significant amounts of unlabeled Fc fragment remained when other Z-M variants were used. Control experiments without Z-M-PyOx showed negligible labeling, indicating minimal background labeling.

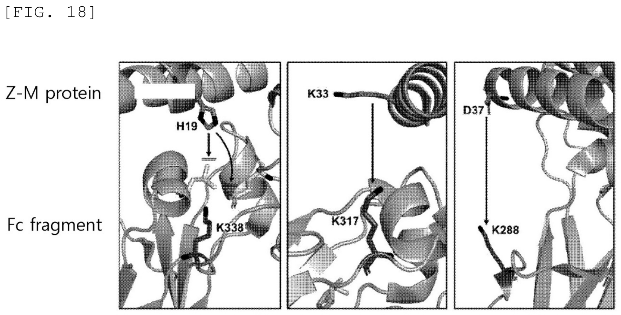

Unlike the Z-DM labeling using Z-AFB, the labeling efficiency of Fc fragment labeling using Z-M did not show a strong correlation with the distance between PyOx and the target Lys. The distance between the PyOx position and Lys was measured in the order of M8 > H19 > D37 > K33, whereas the labeling efficiency was observed in the order of M8 > D37 > K33 > H19 *(see* FIGS. 16 and 17). The most efficient labeling occurred when PyOx was incorporated at the closest site (M8), whereas the second closest site (H19) exhibited the lowest labeling efficiency. This result can be explained by a detailed examination of the complex structural of the Fc fragments and the Z-M protein variants. K338 in the Fc fragment is a residue involved in the labeling reaction when using Z-M_H19AzK-PyOx. In the complex structure, K338 is partially buried by adjacent residues, potentially causing steric hindrance to the labeling reaction *(see* FIG. 18). Thus, the efficiency of the labeling reaction using Z-M_H19AzK-PyOx was lower than that of Z-M_D37AzK-PyOx or Z-M_K33AzK-PyOx despite the shorter distance. This indicates that when selecting PyOx incorporation sites, the distance to the labeled Lys and the surrounding environment of the Lys to be labeled are also important factors.

### 2) Additional Labeling Experiments

Additional labeling experiments were conducted using Z-M_M8AzK-PyOx, which exhibited the highest labeling efficiency in the Fc fragment labeling experiment. In addition to NASA-FL, NASA-Bt *(see* Example 3.6) was used in the Fc labeling experiment, and HPLC analysis confirmed that it exhibited high labeling efficiency similar to that of NASA-FL *(see* FIG. 13).

In addition, Z-M_M8AzK-PyOx_C₆, which contains a slightly longer alkyl chain in the PyOx linker, was prepared and analyzed for labeling efficiency. Z-M_M8AzK-PyOx_C₆ exhibited quantitative labeling efficiency nearly identical to that of Z-M_M8AzK-PyOx. Both variants, Z-M_M8AzK-PyOx and Z-M_M8AzK-PyOx_C₆ exhibited efficient Fc fragment labeling, and therefore, no further optimization of PyOx linker length was performed.

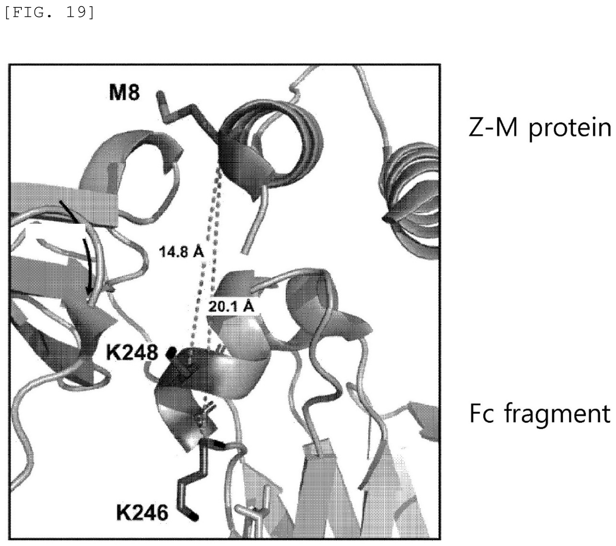

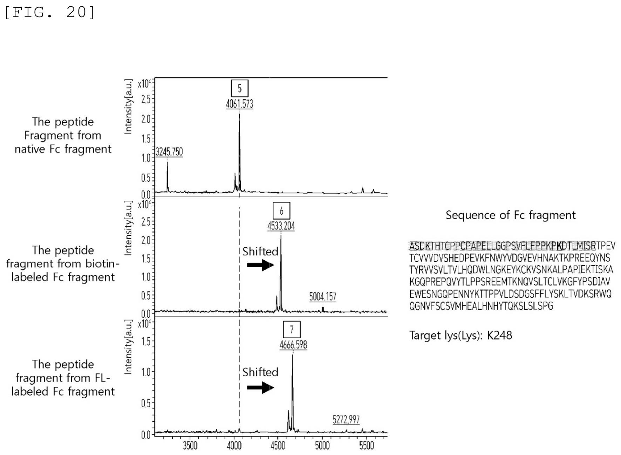

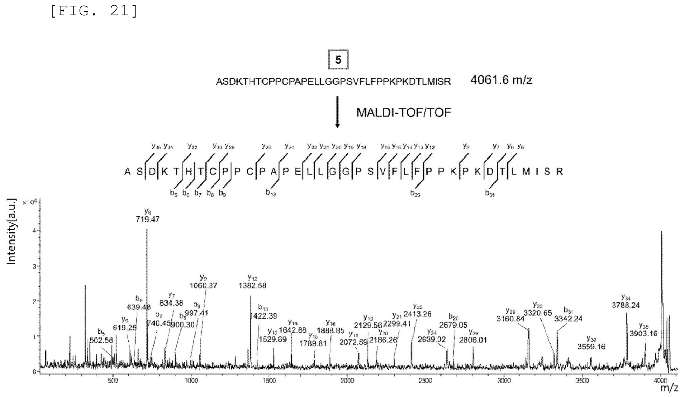

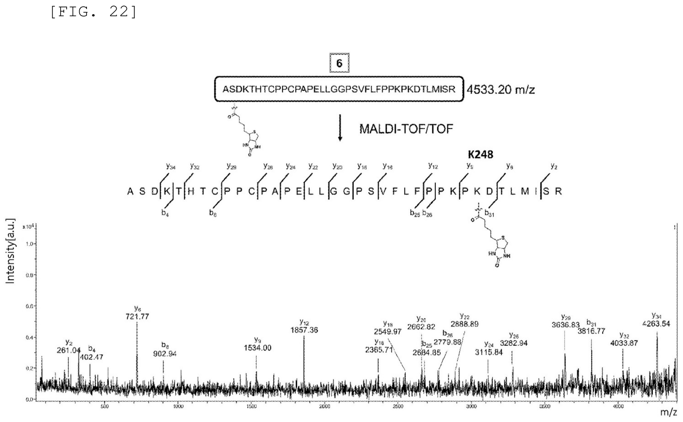

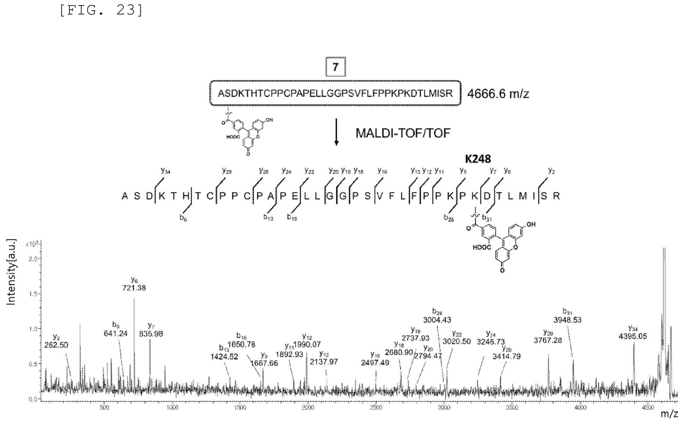

Next, the labeled residues of the Fc fragment labeled using Z-M_M8AzK-PyOx were characterized. The complex structure of the Z-M protein variant and the Fc fragment indicates that the closest Lys to M8 of Z-M in the Fc fragment is K248, and another Lys (K246) is found nearby *(see* FIG. 19). To identify the labeled residue, the labeled Fc fragment was treated with trypsin, and the resulting peptide fragments were analyzed using MALDI-TOF/TOF tandem MS. The peptide fragments containing K248 was clearly shifted to the corresponding labeled fragment for both the FL-labeled and Bt-labeled fragments in MALDI-TOF analysis *(see* FIG. 20). As observed in the HPLC results (FIG. 17), no or negligible unlabeled fragments were detected. In the MALDI-TOF/TOF tandem MS analysis of the peptide fragments, K248 was clearly identified as labeled in the fragment, with no detection of other labeled residues (e.g., K246) *(see* FIGS. 21 to 23).

### 4.6. Labeling Experiment on Intact Trastuzumab

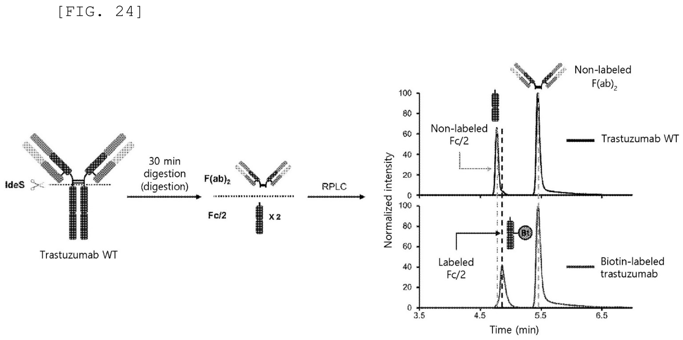

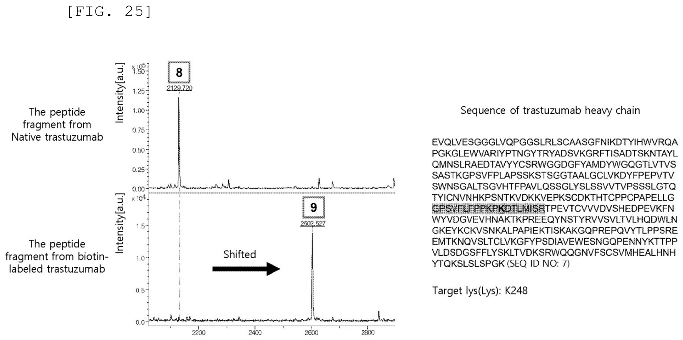

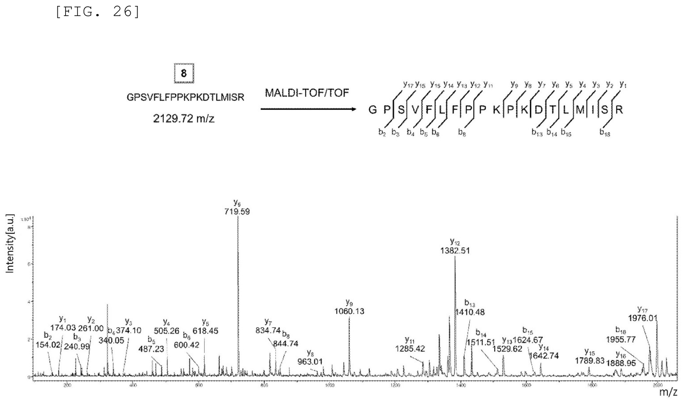

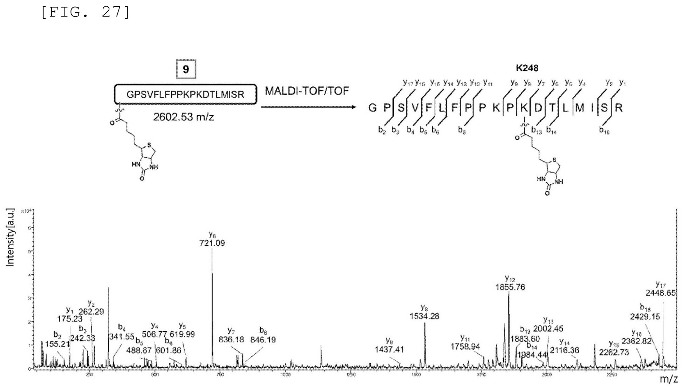

To apply the efficient and selective labeling method of the Fc fragment using Z-M_M8AzK-PyOx to antibody labeling, trastuzumab, which specifically binds to human epidermal growth factor receptor 2 (HER2) and is used in cancer treatment, was employed. Trastuzumab was labeled using Z-M_M8AzK-PyOx and NASA-Bt and analyzed by HPLC. When the whole antibody was analyzed by HPLC, the resolution was insufficient, so the antibody was cleaved into F(ab)₂ and Fc/2 fragments using the IdeS enzyme. As a result, only the Fc/2 peak was found to have shifted, confirming specific labeling for the Fc fragment *(see* FIG. 24). Subsequently, MALDI-TOF/TOF tandem MS analysis revealed biotin labeling at K248, consistent with the Fc fragment results described above *(see* FIGS. 25 to 27).

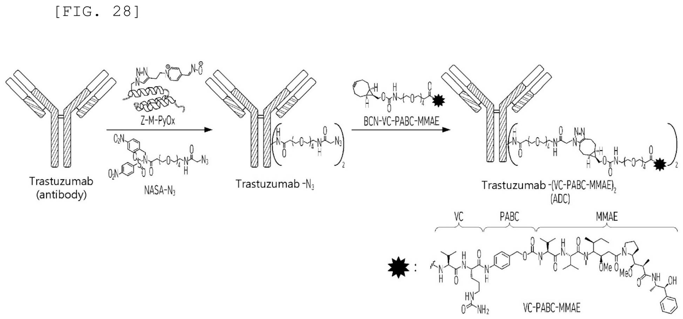

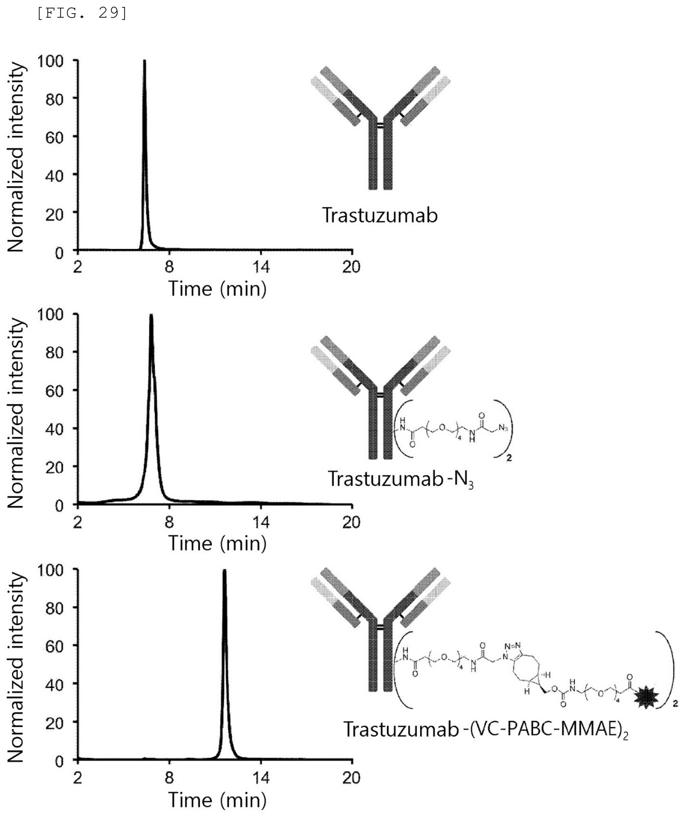

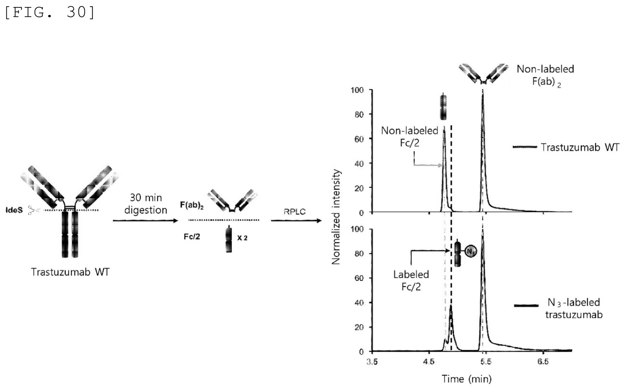

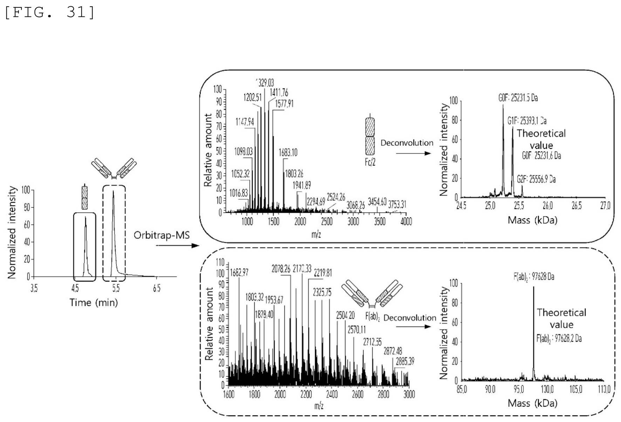

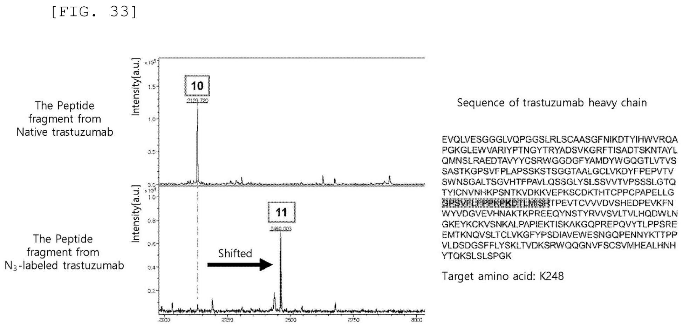

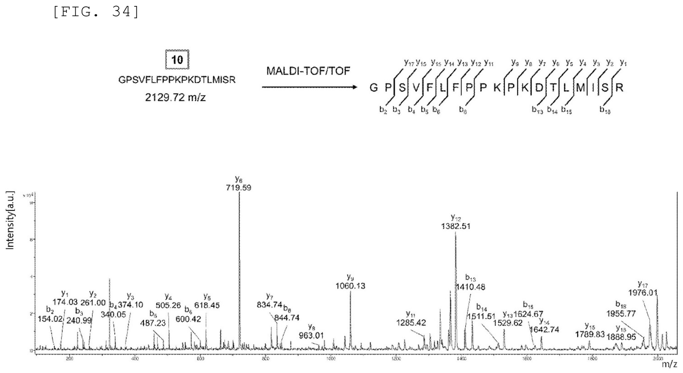

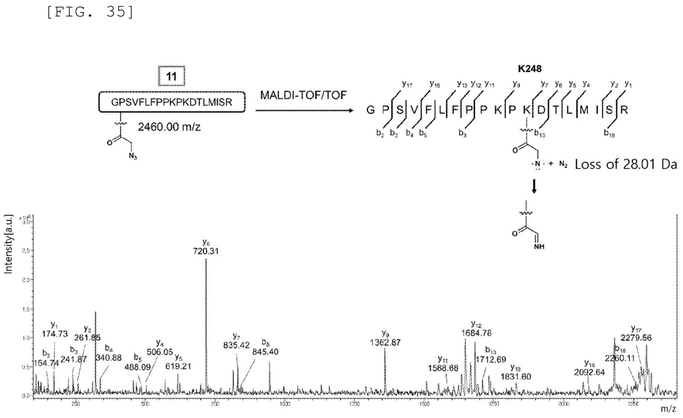

Next, trastuzumab was labeled using a drug for ACD synthesis. An attempt was made to synthesize the ADC using monomethyl auristatin E (MMAE), the most widely used drug among FDA-approved ADCs, through the NASA moiety incorporated in the valine-citrulline-p-aminobenzoyloxycarbonyl (VC-PABC) linker; however, due to the solubility issues of NASA-VC-PABC-MMAE, VC-PABC-MMAE could not be directly introduced into trastuzumab. Instead, NASA-N₃ (*see* Example 3.7) was used to incorporate an azide group onto trastuzumab, and strain-promoted azide-alkyne cycloaddition (SPAAC) was performed to incorporate VC-PABC-MMAE *(see* FIG. 28), using Z-M_M8AzK-PyOx and NASA-N₃ to carry out a trastuzumab labeling reaction. Trastuzumab was efficiently labeled with N₃, but the efficiency was slightly lower than that of NASA-Bt *(see* FIGS. 29 to 32), and MS analysis confirmed that the labeling occurred at the same site (K248) as in Fc fragment labeling *(see* FIGS. 33 to 35).

To incorporate VC-PABC-MMAE onto trastuzumab-N₃, bicyclo[6.1.0]non-4-yne (BCN), which is a modified alkyne group widely used in SPAAC reactions, was used to prepare BCN-VC-PABC-MMAE *(see* Example 3.8 and FIG. 28). SPAAC reactions were performed on trastuzumab-N₃ and BCN-VC-PABC-MMAE to obtain trastuzumab-(VC-PABC-MMAE)₂, which was purified by FPLC to yield a homogeneous trastuzumab-(VC-PABC-MMAE)₂ (*see* FIG. 29).

### 4.7. Cell Viability Assay

To evaluate the cytotoxicity of the synthesized ADC, cell viability assays were conducted using breast cancer cell lines. Three cell lines were used, including two HER2-positive cell lines (SK-BR-3: high; MDA-MB-453: low) and one HER2-negative cell line (MDA-MB-231). The cells were treated with trastuzumab-(VC-PABC-MMAE)₂, and trastuzumab WT and Kadcyla were also tested for comparison.

**[Table 2]**

| Samples | MDA-MB-231 | GI50s (nM) | SK-BR-3 |
|---|---|---|---|
| Kadcyla | >10 | >10 | 0.249±0.043 |
| Trastuzumab WT | >10 | >10 | >10 |
| Trastuzumab-MMAE₂ | >10 | >10 | 0.125±0.065 |

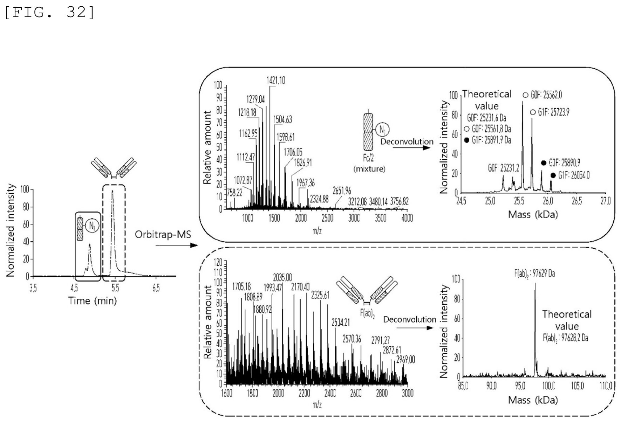

Referring to Table 2 and FIG. 32, the analysis results showed that trastuzumab-(VC-PABC-MMAE)₂ exhibited high potency against HER2-positive SK-BR-3 cells. Furthermore, the ADC exhibited slightly higher efficacy compared to Kadcyla. Conversely, HER2-negative MDA-MB-231 cells exhibited no cytotoxicity, while the HER2-low expressing MDA-MB-453 cells exhibited moderate cytotoxicity.

### 4.8. Binding Affinity Measurement of Trastuzumab-(VC-PABC-MMAE)₂ to HER2 and FcRn

Additionally, the binding affinities of trastuzumab-(VC-PABC-MMAE)₂ to HER2 and FcRn were measured.

**[Table 3]**

| Samples | K_{D} (M) | Kₐ (1/Ms) | K_{d} (1/s) | Rₘₐₓ (RU) | Chi² (RU²) | U-value |
|---|---|---|---|---|---|---|
| Trastuzumab -WT | 3.672x10⁻¹⁰ | 4.343x10⁵ | 1.595x10⁻⁴ | 80.24 03 | 0.256 | 1 |
| Trastuzumab -MMAE₂ | 3.824x10⁻¹⁰ | 4.340x10⁵ | 1.660x10⁻⁴ | 86.64 | 0.155 | 1 |

**[Table 4]**

| Samples | K_{D} (M) | Kₐ (1/Ms) | K_{d} (1/s) | Rₘₐₓ (RU) | Chi² (RU²) | U-value |
|---|---|---|---|---|---|---|
| Trastuzumab -WT | 5.718x10⁻¹⁰ | 3.123x10⁵ | 1.786x10⁻⁴ | 231.7 | 14.1 | 2 |
| Trastuzumab -MMAE₂ | 6.040x10⁻¹⁰ | 2.950x10⁵ | 1.782x10⁻⁴ | 246.6 | 1.42 | 1 |

Referring to Tables 3 and 4, it was confirmed that labeling the antibody with the drug did not affect binding affinity, which indicates that the binding affinity was similar to that of trastuzumab WT. These results demonstrate that the ADC generated using the labeling system of this Example has high cancer cell-specific cytotoxicity and that its binding affinity to FcRn is not impaired, suggesting its potential for future therapeutic applications.

The foregoing description of the present disclosure is provided for illustrative purposes only, and those skilled in the art to which the present disclosure pertains will readily appreciate that the present disclosure can be readily modified into other specific forms without altering the technical spirit or essential features of the present disclosure. Accordingly, the above-described embodiments should be understood as illustrative and not restrictive in any respects. For example, each component described as a single entity may be implemented in a distributed manner, and similarly, components described as distributed may also be implemented in a combined form.

## Claims

1. A conjugation-mediating protein comprising a binding protein into which non-canonical amino acids are incorporated and a pyridinium oxime derivative conjugated to the binding protein.

2. The conjugation-mediating protein of claim 1, wherein the binding protein is a protein derived from protein A, protein G, protein L, or an analogous protein having the same function, all of which are known to bind to antibodies.

3. The conjugation-mediating protein of claim 2, wherein the binding protein is a B-domain variant derived from protein A.

4. The conjugation-mediating protein of claim 3, wherein the B-domain variant is set forth in SEQ ID NO: 1 or SEQ ID NO: 2.

5. The conjugation-mediating protein of claim 1, wherein the non-canonical amino acid is selected from the group consisting of amino acids represented by the following Formulas 1 to 9:

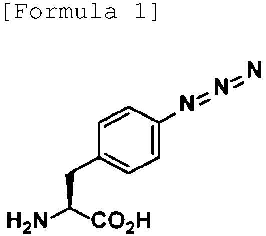

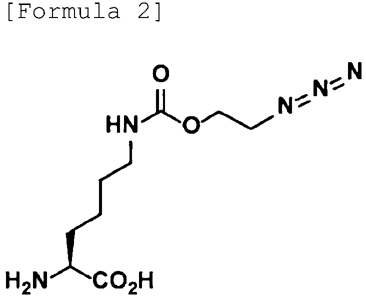

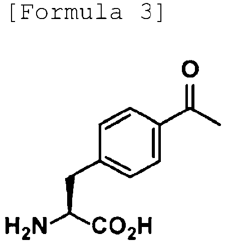

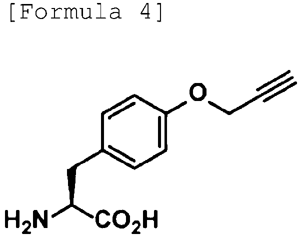

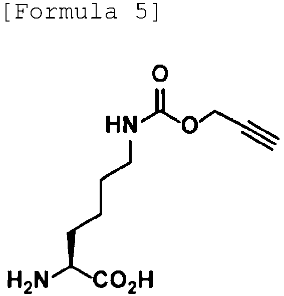

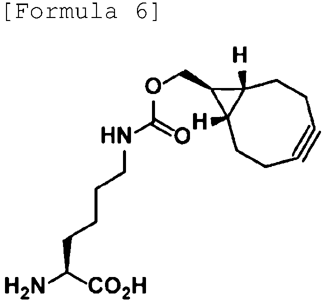

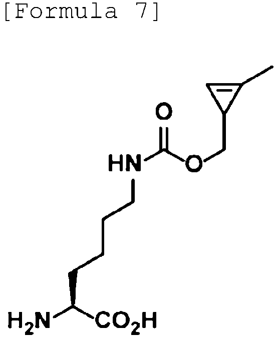

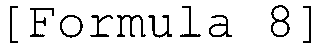

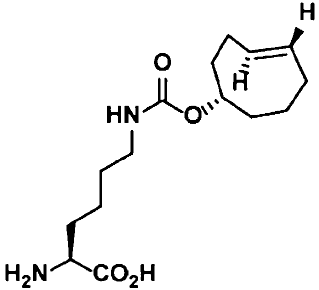

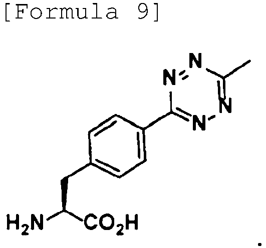

6. The conjugation-mediating protein of claim 1, wherein the non-canonical amino acid is 4-azido-L-phenylalanine or N₆-[(2-azidoethoxy)carbonyl]-L-lysine.

7. The conjugation-mediating protein of claim 1, wherein the non-canonical amino acid is incorporated by substituting at least one amino acid included in the binding protein.

8. The conjugation-mediating protein of claim 1, wherein the pyridinium oxime derivative is represented by the following Formula 1: 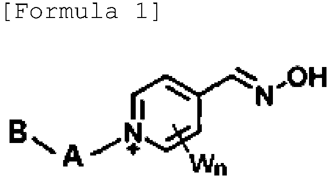 wherein:
A is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 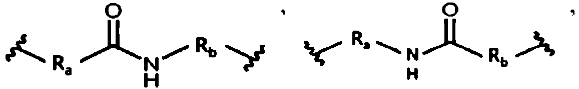 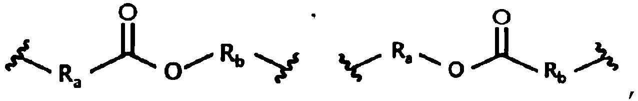 and combinations thereof,
Rₐ and R_{b} are each independently absent or a substituted or unsubstituted C₁-C₁₀ alkylene group, or a substituted or unsubstituted C₂-C₁₀ polyethylene glycol group,
B is selected from the group consisting of 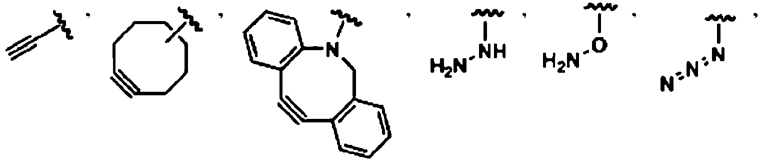 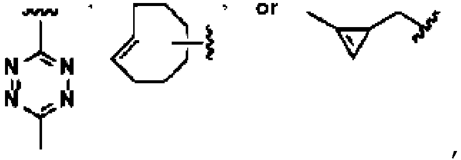
W is -F, -Cl, -Br, -NO₂, or -CF₃, and
n is an integer from 0 to 4.

9. The conjugation-mediating protein of claim 8, wherein B of the pyridinium oxime derivative of Formula 1 reacts with and binds to a terminal functional group of the non-canonical amino acid.

10. A method for labeling an antibody, comprising:
a first step of binding the conjugation-mediating protein of any one of claims 1 to 9 to a native antibody;
a second step of reacting the conjugation-mediating protein with a probe-bound *N*-alkylsulfonamide derivative to form an active ester intermediate; and
a third step of reacting lysine contained in an Fc domain of the native antibody with the intermediate to form an amide bond.

11. The method of claim 10, wherein the probe-bound N-alkylsulfonamide derivative is represented by the following Formula 2: 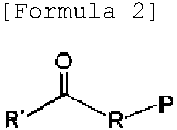 wherein:
R' is 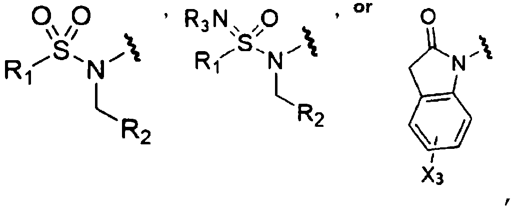
R is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 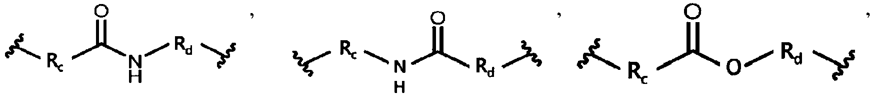 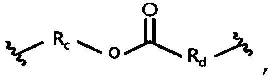 and combinations thereof,
R_{c} and R_{d} are each independently absent or a substituted or unsubstituted C₁-C₁₀ alkylene group, or a substituted or unsubstituted C₂-C₁₀ polyethylene glycol group,
R₁ is selected from CF₃ or 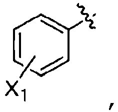
R₂ is selected from CF₃, 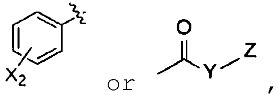
R₃ is H or a linear or branched C₁-C₅ alkyl group,
P is selected from 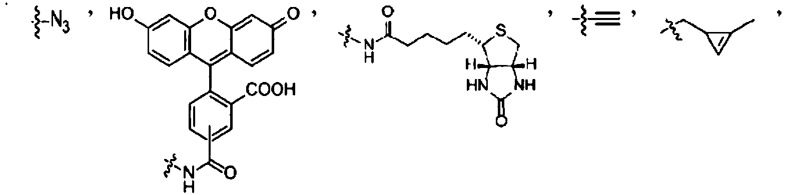 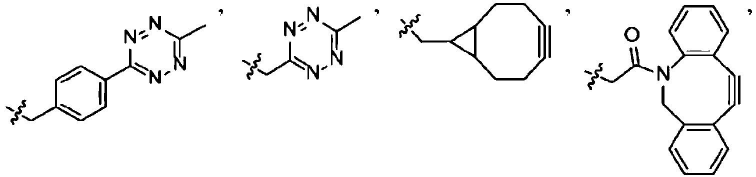 or 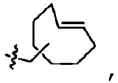
X₁, X₂, and X₃ substitute one or more hydrogen atoms on the phenyl group, and are each independently F, Cl, Br, CF₃, or NO₂,
Y is O or NH, and
Z is a linear or branched C₁-C₂₀ alkyl group which is substituted or unsubstituted with -NRₑR_{f},
wherein Rₑ and R_{f} are each independently a C₁-C₅ alkyl group, or a C₄-C₁₀ cyclic alkyl group linked to each other, and may optionally contain heteroatoms selected from N, S, or O or not.

12. The method of claim 10, wherein the probe-bound *N-*alkylsulfonamide derivative is selected from the group consisting of *N*-alkylsulfonamide-fluorescein (NASA-FL), *N-*alkylsulfonamide-biotin (NASA-Bt), and *N*-alkylsulfonamide-N₃.

13. A method for the site-specific synthesis of antibody-drug conjugates, comprising:
a first step of binding the conjugation-mediating protein of any one of claims 1 to 9 to a native antibody;
a second step of reacting the conjugation-mediating protein with a probe-bound *N*-alkylsulfonamide derivative to form an active ester intermediate;
a third step of reacting lysine contained in an Fc domain of the native antibody with the intermediate to form an amide bond; and
a fourth step of reacting the probe with a drug to label the drug.

14. The method of claim 13, wherein the probe-bound *N-*alkylsulfonamide derivative is represented by the following Formula 2: 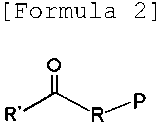 wherein:
R' is 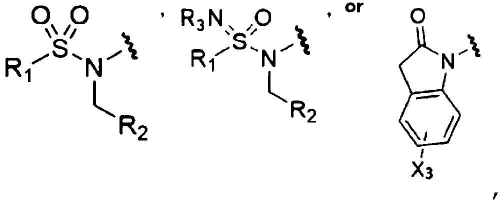
R is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 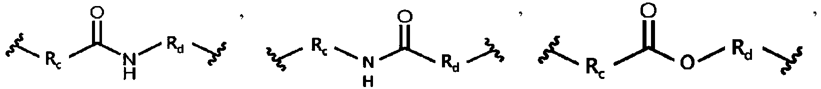 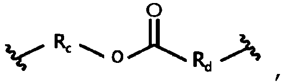 and combinations thereof,
R_{c} and R_{d} are each independently absent or a substituted or unsubstituted C₁-C₁₀ alkylene group, or a substituted or unsubstituted C₂-C₁₀ polyethylene glycol group,
R₁ is selected from CF₃ or 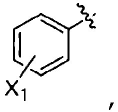
R₂ is selected from CF₃, 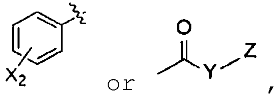
R₃ is H or a linear or branched C₁-C₅ alkyl group,
P is selected from 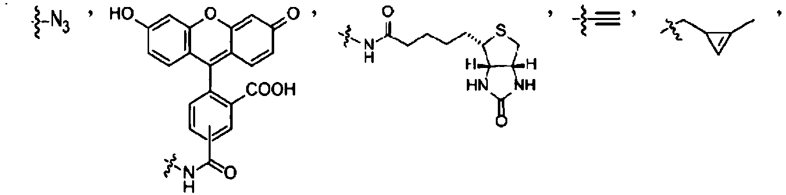 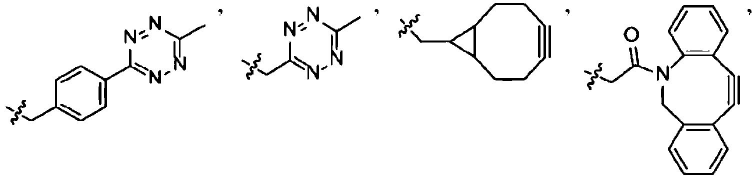 or 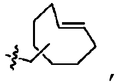
X₁, X₂, and X₃ substitute one or more hydrogen atoms on the phenyl group, and are each independently F, Cl, Br, CF₃, or NO₂,
Y is O or NH, and
Z is a linear or branched C₁-C₂₀ alkyl group which is substituted or unsubstituted with -NRₑR_{f},
wherein Rₑ and R_{f} are each independently a C₁-C₅ alkyl group, or a C₄-C₁₀ cyclic alkyl group linked to each other, and may optionally contain heteroatoms selected from N, S, or O or not.

15. The method of claim 13, wherein the probe-bound *N-*alkylsulfonamide derivative is selected from the group consisting of *N*-alkylsulfonamide-fluorescein (NASA-FL), *N-*alkylsulfonamide-biotin (NASA-Bt), and *N*-alkylsulfonamide-N₃.

16. The method of claim 13, wherein the drug is a drug or a drug comprising a cleavable linker.

17. The method of claim 16, wherein the drug is selected from the group consisting of monomethyl auristatin E (MMAE), Maytansinoids, Calicheamicin, exatecan, SN-38, monomethyl auristatin F (MMAF), and derivatives thereof.

18. A method for the site-specific synthesis of antibody-drug conjugates, comprising:
a first step of binding the conjugation-mediating protein of any one of claims 1 to 9 to a native antibody;
a second step of reacting the conjugation-mediating protein with a drug-bound *N*-alkylsulfonamide derivative to form an active ester intermediate; and
a third step of reacting lysine contained in an Fc domain of the native antibody with the intermediate to form an amide bond.

19. The method of claim 18, the drug-bound *N-*alkylsulfonamide derivative is represented by the following Formula 3: 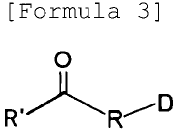 wherein:
R' is 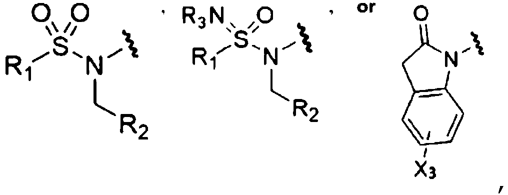
R is absent, or is selected from the group consisting of a substituted or unsubstituted C₁-C₁₀ alkylene group, a substituted or unsubstituted C₅-C₁₀ cyclic alkylene group, a substituted or unsubstituted C₅-C₁₀ phenylene group, a substituted or unsubstituted C₂-C₁₀ polyethylene glycol group, a C₁-C₁₀ alkylene group containing heteroatoms selected from N, O, or S, a C₅-C₁₀ cyclic alkylene group containing heteroatoms selected from N, O, or S, a C₅-C₂₀ phenylene group containing heteroatoms selected from N, O, or S, 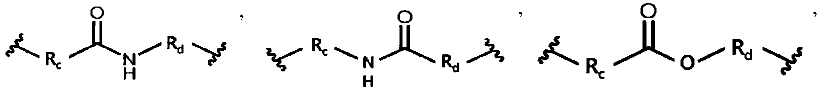 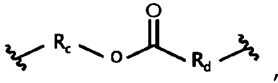 and combinations thereof,
R_{c} and R_{d} are each independently absent or a substituted or unsubstituted C₁-C₁₀ alkylene group, or a substituted or unsubstituted C₂-C₁₀ polyethylene glycol group,
R₁ is selected from CF₃ or 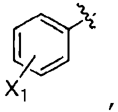
R₂ is selected from CF₃, 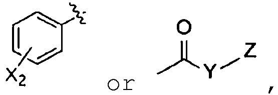
R₃ is H or a linear or branched C₁-C₅ alkyl group,
D is a drug or a drug comprising a cleavable linker,
X₁, X₂, and X₃ substitute one or more hydrogen atoms on the phenyl group, and are each independently F, Cl, Br, CF₃, or NO₂,
Y is O or NH, and
Z is a linear or branched C₁-C₂₀ alkyl group which is substituted or unsubstituted with -NRₑR_{f},
wherein Rₑ and R_{f} are each independently a C₁-C₅ alkyl group, or a C₄-C₁₀ cyclic alkyl group linked to each other, and may optionally contain heteroatoms selected from N, S, or O or not.

20. The method of claim 19, wherein the drug is selected from the group consisting of monomethyl auristatin E (MMAE), Maytansinoids, Calicheamicin, exatecan, SN-38, monomethyl auristatin F (MMAF), and derivatives thereof.
